# EUROPEAN PATENT SPECIFICATION

(11) **EP 3 038 938 B1**
(45) Date of publication and mention of the grant of the patent: **17.10.2018**
(21) Application number: 14840874.3
(22) Date of filing: 29.08.2014
(51) Int. Cl.: B65D 33/16, B65D 33/17, B65D 30/10

(54) **METHOD OF FORMING TAMPER RESISTANT PACKAGE**
VERFAHREN ZUR HERSTELLUNG MANIPULATIONSSICHERER VERPACKUNG
PROCÉDÉ DE FABRICATION D'EMBALLAGE INVIOLABLE

(30) Priority: 30.08.2013 US 201361872148 P
(43) Date of publication of application: 06.07.2016
(73) Proprietor: Kimberly-Clark Worldwide, Inc., Neenah, Wisconsin 54956 (US)
(72) Inventor: TOMSOVIC, Charles R., Omro, Wisconsin 54963 (US); FRANGER, Stephen D., Neenah, Wisconsin 54956 (US); GOTTVALD, Martin, Appleton, Wisconsin 54913 (US); O'BRIEN, Sarah Austin, Neenah, Wisconsin 54956 (US); SCHMIDT-FORST, Alexander Manfred, Appleton, Wisconsin 54913 (US)
(74) Representative: Dehns
(86) International application number: PCT/US2014/053504
(87) International publication number: WO 2015/031811

(56) References cited:
- JP-A- 2005 289 396
- JP-A- 2010 018 343
- JP-A- 2010 163 192
- JP-A- 2013 052 927
- KR-A- 20120 098 268
- KR-A- 20120 098 268
- KR-A- 20130 077 154
- US-A- 4 648 513
- US-A- 4 898 280
- US-A- 5 611 627
- US-A- 5 851 071
- US-A1- 2006 188 180
- US-A1- 2008 105 679
- US-A1- 2008 152 264
- US-A1- 2008 292 222
- US-A1- 2009 317 578
- US-A1- 2011 132 976

## Description

### FIELD

The present disclosure relates to methods of forming tamper resistant packaging, and in particular, tamper resistant packaging for absorbent personal care articles, such as tampons.

### BACKGROUND

Depending on the type of absorbent personal care articles to be stored in bag packaging (such as in polymer-based or foil-based film bags), it has become commonplace for such packaging to include resealable structures such as adhesive-based strips, interlocking pieces, and/or zipper-like structures that enable manual opening and closing of the packaging. One of the more prevalent types of interlocking pieces can be generally described as interlocking rib and groove structures that are positioned between opposed walls of the packaging, although the interlocking pieces can also include hook and loop-style structures instead of the interlocking rib and groove structures. Application of pressure on the opposed interlocking groove and rib structures along their length dimension at the same time causes the rib to removably sit within the groove, thereby creating a temporary seal for the opening in the packaging. Such structures imitate a zipper-type movement during the sealing and unsealing operation, although the interlocking teeth common to a traditional zipper are missing. Such interlocking, zipper-like features are typically placed either immediately along or adjacent edges that define the opening of the bag packaging and allow for selective opening and resealing of the opening of the bag packaging. The zipper-like structure may also include a sliding mechanism which assists in locking and unlocking (i.e., engaging and disengaging) the interlocking pieces to selectively seal and unseal the bag's opening.

The interlocking pieces or zipper-like structure facilitate a user's (e.g., a consumer) ability to selectively remove one or more of the articles from the packaging without destroying the packaging. Such resealable packaging has been used for a wide array of consumer goods, such as for the storage of produce, sandwiches, disposable razors, and tampons. Traditional resealable or zippered bags have an opening defined by straight edges adjacent and/or parallel to the resealable or zipper-like structure for ease of manufacture. The bag is either molded in place to have straight edges, or cut with a straight cutting edge adjacent the resealable/zipper-like feature. As a result of bag manufacturing processes, the straight edges that define the opening of the bag often curl towards each other making their separation by the user during use of the bag more difficult.

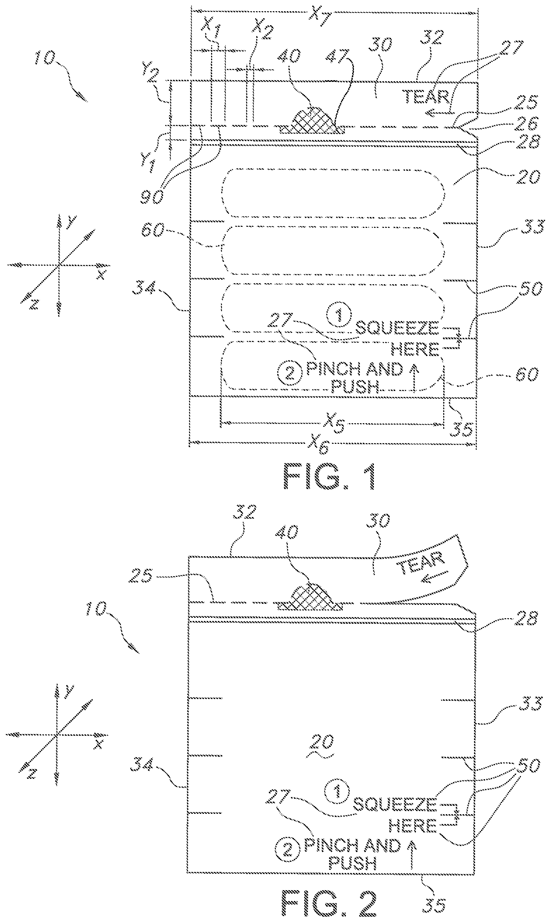

The description of plastic containers in United States Patent Application Publication No. 2008/0105679 to Ballard exemplifies resealable packaging. The packaging described in Ballard additionally includes offset opening tabs along the opening edges, adjacent the interlocking, zipper-like feature. Such offset tabs are not centered with respect to one another, and allow for the easy grasping and separation of the opening edges (to form an easy opening feature). See in particular, Fig. 2 of Ballard. As shown in the figure, such offset tabs are staggered with respect to one another, along the full opening edge of both bag walls. Without such tabs, it is often difficult to separate the normally straight edges of the bag opening, adjacent the zipper-like feature. The offset tabs provide material extensions of the bag walls, which extensions are visually and more tactilely apparent, and easier to separate than the straight edges. Additionally, since polymer and foil films tend to adhere to like materials (like the opposing walls of a bag), the offset tabs offer an additional means of separating adhering walls from one another, when static electricity or other forces might keep the bag edges together.

Offset tabs are further illustrated in United States Patent No. 5,683,340 to Belias, which illustrates numerous offset tab designs for bag opening edges, and also in United States Patent Application Publication No. 2011/268373 to Polland, which illustrates a single pair of noncentered tabs along the edges of a bag opening. As with straight edges, the manufacture of such offset tabs on a bag typically involves either the premolding of the edges to a particular shape, or the cutting of the edges during manufacture. However, given the nature of the tab protuberance, such cutting is often accomplished by a non-straight edge shaped cutting die, or a more elaborate cutting apparatus. The tab feature can therefore add a degree of manufacturing complexity not seen in bags having only straight edges. Even with the use of offset tabs, such packaging, while being resealable, is not tamper resistant.

It has been known to use tamper resistant features in connection with resealable bags having openings with straight edges. For example, such tamper resistant features have been used on bags which store consumable items that may pose consumer safety hazards if exposed to germs before use or if exposed to the environment ahead of use. Such tamper resistant features are also placed on such bags to signal intentional tampering before article purchase or to assure a user of a clean or sterile environment along the interior surface of the bag prior to use. See for example, the disclosure in United States Patent Application Publication No. 2011/0158565 to Hellming et al.

Such tamper resistant and resealable bags have been used to store items as diverse as prewashed salad greens, disposable razors, and tampons and are usually presented to the consumer with the resealable feature sealed. The tamper resistant packaging often includes a safety seal adjacent the bag opening (and adjacent the resealable structure). The safety seal is sealed until a user intentionally ruptures, cuts, or rips it. For instance, a pull, tear or cutting region on the safety seal is frequently identified for the user to remove prior to opening an interlocking, zipper-like feature. Once the safety seal is either removed or altered, the resealable structure is exposed for the user to unseal and, thus, open the bag packaging. See for example, the disclosure in Hellming above and United States Patent No. 7,134,788 to Hsiang. The removal or altering of the safety seal is often done in a single step, such that multiple pieces of packaging do not have to be separately removed by consumers, and extra waste pieces are eliminated. While such single step removal is initially easier for the consumer, the tear-away portion and resulting bag opening edges are often straight parallel edges or slightly uneven, again leading to difficulty in opening the bag at the resealable portion.

Therefore, there is a need for tamper resistant polymer film or foil bags that allow for easy opening, but which also provide for the safekeeping of items stored within the bag until the bag is intentionally opened by a user. Additionally, there is a need for easy opening and resealable bags that signal a user when such bag has previously been opened by someone other than the intended user, or has been otherwise structurally compromised.

Documents JP2013/052927, JP2010/163192, JP2010/018343 and KR2012/0098268 disclose different tamper resistant bags that have removable strip portions and resealable features, as well as tab features to aid with opening. These bags are not contemplated for storing absorbent articles and there is scope to improve on their disclosed designs. Documents US 2011/0132976 A1 and US 4,648,513 disclose methods of wrapping an absorbent article.

Often, users find it challenging to dispense individual absorbent articles from storage bags used to store personal care absorbent articles. When such bags are opened, either in a public or private setting, it is often difficult for the user to retrieve a single desired product inconspicuously from the package. Since typically only one product is used at a time, it is desirable for a user to be able to remove a single product from a package without multiple products falling from the package. However, the neck of the bag opening may be too narrow to allow a user to easily insert his/her hand into the bag so as to select the single desired product. Conversely, the neck of the bag may be too wide to easily allow the pouring of an individual article (or the selection of one article without contacting others in the bag). If the bag is tilted over to pour the article from the bag opening, often multiple unneeded articles are dispensed together rather than the desired single article. Sometimes individually wrapped absorbent products are tightly packed within an outer package, and it is quite difficult to withdraw only one at a time. For articles involving personal body care, the unintentional contact with such unused and unneeded articles is not favored, so as to preserve the cleanliness of such articles until actual use is desired. Therefore, there is a need for easy dispensing features that may be used in conjunction with easy opening, tamper resistant and resealable packaging to facilitate the removal of a single article from a package at any given time.

### SUMMARY

According to a first aspect of the present invention, a method is provided for forming a resealable and tamper resistant bag for enclosing at least one absorbent article as claimed in claim 1.

According to a second aspect of the present invention, a method is provided for forming a plurality of resealable and tamper resistant bags for enclosing absorbent articles therein as claimed in claim 4.

In an example of a bag a resealable and tamper resistant bag has a longitudinal direction, a transverse direction, a depth direction, and at least four peripheral side edges. The bag generally comprises a front wall including a first resealable feature, and a back wall including a second resealable feature adapted to form a seal with the first resealable feature of the front wall. The front and back walls are sealingly connected along at least a pair of the sides to define at least part of a holding portion of the bag. A sealed tamper resistant strip portion is formed integrally with the holding portion and disposed adjacent the first and second resealable features of the front and back walls. At least one seam is disposed between the holding portion and the strip portion for allowing the strip portion to be selectively removed, at least in part, from the holding portion by a user. At least one tab is defined by the seam to facilitate the user moving the first and second resealable features from a sealed position to an unsealed position. The seam is provided by perforations and at least one tab is defined by one or more extended perforations which traverse at least a portion of the periphery of the at least one tab.

In another example a resealable and tamper resistant bag has a longitudinal direction, a transverse direction, and a depth direction, and at least four peripheral side edges. The bag includes at least first and second opposing substantially planar panels, sealingly connected along at least a pair of sides, forming at least part of a bag holding portion. The bag further includes a pair of resealable features either integrally formed or non-integrally formed, one each on or in the planar panels. An integrally formed and sealed tamper resistant strip portion is adjacent the resealable features. At least one separating seam defines the sealed tamper resistant strip portion. The seam defines at least one easy opening tab on the bag. In an alternative embodiment, the resealable and tamper resistant bag includes four or more planar panels. In still another alternative embodiment, the resealable and tamper resistant bag includes five or more planar panels. In yet another alternative embodiment, the resealable and tamper resistant bag includes a pair of resealable features that are selected from the group consisting of interlocking groove and rib structures, adhesive strips, hook and loop structures, materials having varying coefficients of friction, magnetic fastening systems, and zipper-like features. In another alternative embodiment, the tamper resistant strip portion is removable from the resealable and tamper resistant bag. In still another alternative embodiment, the tamper resistant strip portion includes a folded easy opening tab structure. In such an embodiment, the tamper resistant strip portion may or may not be removable from the bag.

In still another alternative example the separating seam is formed from perforations that are selected from the group consisting of perforated dashes, perforated lines and perforated dots. In another alternative embodiment, the seam includes both perforations and weakened areas, with the perforations situated at least around a portion of the periphery of the at least one easy opening tab. In yet another example of the bag, the bag includes at least two easy opening tabs. Such tabs may be on the same wall or different walls of the bag. Further such tabs may be integral or non-integral with at least one of the bag walls.

In another alternative example the bag includes single or multiple easy opening tabs extending along the entire transverse direction of the bag. In yet another alternative example, the easy opening tab(s) includes a folded portion. In a further alternative embodiment, the easy opening tab is supported by a strengthening agent selected from the group consisting of a strengthening coating, additional basis weight of tab-forming material, and adhesive patches of strengthening material. In another example, the bag includes at least one dispensing indicator along at least one planar panel for facilitating the dispensing of an individual article that may be contained in the bag. Such dispensing indicator may be made of words, symbols or a combination of the two. In a further alternative example, the dispensing indicator is positioned on at least one planar panel in a direction parallel with the resealable features. In a further alternative example, the dispensing indicator is positioned on at least one planar panel in a direction perpendicular with the resealable features. In still a further alternative example, an integrally formed and sealed tamper resistant strip portion includes a removal indicator for facilitating the removal of the strip portion along the seam. Such strip may also include a tear initiating structure such as a cutout.

In another alternative example, the resealable and tamper resistant bag includes stacked, individually wrapped personal care absorbent articles that are contained in the bag, the articles are stacked in either a direction parallel with or perpendicular to the resealable features and further, at least one of the planar panels includes at least one dispensing indicator.

In another alternative example, the resealable and tamper resistant bag includes at least two easy opening tabs, the tabs being defined by two seams, one seam being present within each planar panel, with each of the tabs being separately defined by each of the two seams. The seams are generally aligned with each other, with the exception of the seam-defined tabs, the tabs being off-center with respect to each other along the bag Z direction.

In an alternative example, the resealable and tamper resistant bag includes tabs that are of generally semicircular shapes and include peaks, and the peak- to -peak distance is between about 1 and 75 mm, alternatively between about 5 and 30 mm. The peak to peak distance is either between adjacent peaks on the same wall, or on different, opposed walls. In still another alternative example, the at least one tab(s) has a width at the base of between about 5 and 75 mm in the transverse direction. In another alternative example, the at least one tab has a height of between about 5 and 50 mm. In still a further alternative example, the tabs have a percent surface area overlap in the Z direction of between about 0 and 90%.

In an alternative example, the resealable and tamper resistant bag includes tabs that are separated along the transverse direction by a distance of between about 0 and 50 mm. In an alternative example, the resealable and tamper resistant bag includes a separating seam having at least a straight seam length portion along the bag transverse direction that is parallel with the resealable features, alternatively two straight seam length portions. In an alternative example, the resealable and tamper resistant bag includes a separating seam that includes no straight seam length portion that is parallel with the resealable features. An example method for forming a resealable and tamper resistant bag includes the steps of a) providing a planar substrate or sheet material having a machine direction and a cross-machine direction, the planar substrate having machine direction and cross-machine direction edges; b) attaching or otherwise adhering a pair of resealable features adjacent to opposing cross-machine direction edges of the planar substrate, and along the same surface of the planar substrate, the resealable features running along the machine direction of the planar substrate; c) perforating two lines or otherwise providing two lines of weakness along the planar substrate machine direction, such that the perforation lines or lines of weakness are each situated the same distance laterally beyond the resealable features towards the opposing cross-machine direction edges of the planar substrate, the perforation lines or lines of weakness including along their lengths at least two opposing tab portions, the tab portions offset from one another along the machine direction; d) folding the planar substrate along a fold line situated equidistant from the resealable features, thereby aligning the resealable features; e) cutting the planar substrate at least along the cross-machine direction; f) bonding or otherwise affixing the machine and cross-machine direction edges of the planar substrate, such that the resealable features are aligned with one another into a finished bag.

In one example, the folding step is in the cross-machine directional shape of a W. In another suitable example, the folding step is in the cross-machine directional shape of a V. In an alternative example, the resealable features are added to the planar substrate following the perforating or weakening step c). Other suitable examplesof the method include the steps of adding at least one further pair of resealable features along the planar substrate machine direction and/or adding at least a discrete resealable opening within the planar substrate. In one suitable example, the perforating step is conducted with a barrier plate adjacent the planar substrate.

In one suitable example, the method further includes the step of providing dispensing indication means along the planar substrate wherein the dispensing indication means is either parallel to the resealable features, perpendicular to the resealable features, or a combination of such. Suitably, the perforation lines or otherwise weakened lines can be separated from the resealable features along the cross-machine direction, by between about 1 and 50 mm.

According to an embodiment of the present invention, a method for forming a resealable and tamper resistant bag for enclosing at least one absorbent article generally comprises moving a planar substrate in a machine direction. The planar substrate has two opposite edges. A first resealable feature is attached to the substrate generally adjacent one of the edges, and a second resealable feature is attached to the substrate in spaced relationship to the first resealable feature and generally adjacent the other edge. The first and second resealable features are configured to sealingly engage. A first separating seam is formed on the substrate between the first resealable feature and the respective edge of the substrate. The first separating seam defines a first tab. A second separating seam is formed on the substrate between the second resealable feature and the respective edge such that the first and second separating seams are generally the same distance from the respective first and second resealable features. The second separating seam defines a second tab wherein the second tab is offset from the first tab. At least one absorbent article is placed in contact with the substrate. The substrate is folded and sealed to define the resealable and tamper resistant bag having the at least one absorbent article enclosed therein.

According to an alternative embodiment of the present invention, a method for forming a plurality of resealable and tamper resistant bags for enclosing absorbent articles therein generally comprises directing, in a machine direction, a continuous web comprising a plurality of interconnected segments suitable for the construction of a plurality of the bags. The plurality of segments are interconnected in the cross-machine direction. At least one continuous separating seam is formed on the web using a laser. The separation seam extends across the plurality of interconnected segments and defines at least one tab on each of the segments. The continuous web is cut in the cross-machine direction to separate the plurality of interconnected segments from the web to form a plurality of discrete segments. At least one absorbent article is associated with each discrete segment. Each of the discrete segments is folded and sealed to enclose the at least one absorbent article.

### BRIEF DESCRIPTION OF THE DRAWINGS

FIGURE 1 is a front view of one suitable example of an easy opening, tamper resistant and resealable bag package filled with articles.
FIGURE 2 is a front view of the bag package of FIGURE 1 with a removable strip portion partially removed.
FIGURE 3 is a front view of the bag package of FIGURE 1 with the removable strip portion totally removed.
FIGURE 4 is a fragmentary front perspective view of the bag package of FIGURE 1 with a bag opening visible after a resealable feature has been unsealed and the removable strip portion removed.
FIGURE 5 is a front view of another suitable example of the easy opening, tamper resistant and resealable bag package.
FIGURE 5A is a side view of the bag package of FIGURE 5.
FIGURE 5B is a fragmentary front view of another example of perforation lines (or other lines of weakness) defining easy opening tabs.
FIGURE 5C is a fragmentary front view of still another example of perforation lines (or other lines of weakness) defining easy opening tabs.
FIGURE 5D is a fragmentary front view of still another example of perforation lines (or other lines of weakness) defining easy opening tabs.
FIGURE 5E is a fragmentary front view of still another example of perforation lines (or other lines of weakness) defining easy opening tabs.
FIGURE 5F is a fragmentary front view of yet another example of perforation lines (or other lines of weakness) defining easy opening tabs.
FIGURE 5G is a fragmentary front view of another example of easy opening tabs of a resealable bag package.
FIGURE 5H is another view of the tabs of FIGURE 5G.
FIGURE 6 is a front view of another suitable example of the easy opening, tamper resistant and resealable bag package of FIGURE 5.
FIGURE 7 is a top perspective of the bag package of FIGURE 1, illustrating the manual dispensing of one article from the packaging using squeeze indicator lines, once the resealable feature has been unsealed and the bag has been opened.
FIGURE 8 is a view of another suitable example of an article filled bag package such as that shown in FIGURE 5, illustrating the manual dispensing of one article from the packaging using indicator lines and directional verbiage.
FIGURE 9 is a perspective of another suitable example of the bag package of FIGURE 1 illustrating multiple side panels in the bag construction.
FIGURE 10 is a fragmentary front view of another example of the bag package of FIGURE 1, in which the bag front wall includes a perforated seam different from the perforated seam of the bag back wall.
FIGURE 11 is a fragmentary front view of another suitable example of the bag package of FIGURE 1, in which the bag includes a seam on a top edge of the tamper resistant strip portion.
FIGURE 11A is an enlarged view of a portion of the bag package shown in FIGURE 11 showing a movable tab on the seam of the top edge.
FIGURE 11B is an enlarged view of a portion of the bag package shown in FIGURE 11 showing the opened movable tab on the seam of the top edge.
FIGURE 11C is a front view of the bag package of FIGURE 11 with the movable tab in an opened position.
FIGURE 11D is a top perspective of the bag package of FIGURE 11 with the tab being used to separate the tamper resistant strip portion walls.
FIGURE 11E is a further top perspective of the bag package of FIGURE 11 with the walls of the tamper resistant strip portion being separated to expose and open the resealable features.
FIGURE 12 is a fragmentary front view of another suitable example of the bag package of FIGURE 1 including two non-aligned seams on the tamper resistant strip portion, with a strengthening agent attached to one or both of the front and back bag walls.
FIGURE 12A is a fragmentary view of the process of manually removing the tamper resistant strip portion from the bag package shown in FIGURE 12.
FIGURE 12B is a fragmentary view of the process of manually removing the tamper resistant strip portion from the bag package shown in FIGURE 12A.
FIGURE 12C is a top view of the bag package shown in FIGURE 12B with the tamper resistant strip portion removed, and an adhesive decal attached as the strengthening agent.
FIGURE 12D is a fragmentary top view of the process of opening the bag package shown in FIGURE 12 once the tamper resistant strip portion has been removed and the resealable features have been exposed.
FIGURES 13A-13G illustrate one suitable production method for making bag packages from planar sheet materials.
FIGURES 14A-14K illustrate another suitable production method for making bag packages.
FIGURE 15 is a fragmentary front view of still another suitable example of the bag package of FIGURE 1, in which the bag front wall includes a perforated seam different from the perforated seam of the bag back wall as a result of a barrier plate used in bag formation.
FIGURE 16 is a schematic illustrating another suitable production method for making bag packages.
FIGURE 17 is a top plan view of a continuous web exiting a laser station during the production method seen in FIGURE 16.
FIGURE 18 is a side cross-sectional view of the continuous web being cut at the laser station.
FIGURES 19 and 20 are photographs showing portions of suitable examples of bag packages having separating seams and associated tabs formed thereon using a targeted laser scribing technique.
FIGURE 21 illustrates suitable exemplary cutting patterns for forming separating seams with a targeted laser scribing technique.

### DETAILED DESCRIPTION OF THE DRAWINGS

It has been found that one or more offset tabs incorporated in a resealable bag package enable a user (e.g., a consumer) to easily open the resealable bag package. When a tamper resistant (or tamper evident) strip portion is removed from the bag package, the offset tabs can be left in place along the top edge of the bag package to facilitate separation of resealable features. In one suitable embodiment, the offset tabs are strengthened or configured to prevent accidental removal of the tabs with the tamper resistant strip portions. In other embodiments, at least a portion of the tamper resistant strip portion remains in place when it is opened, and the offset tabs remain in place with the remaining portion of the tamper resistant strip portion to assist in the opening of the resealable features. Additionally, selective placement of dispensing indicator marks along at least one direction of the bag package can ease the dispensing of single absorbent articles contained in the bag package.

For purposes of the following description, "like" numbers may be used to indicate "like" or similar features of the various packages disclosed herein. It should be recognized that for the purposes of this application, the term "resealable" is not limited to interlocking rib and groove, or zipper-like structural features. For example, resealable technology can also include materials having different coefficients of friction, hook and loop type fastening systems, and pressure sensitive adhesive arrangements. The term "resealable feature" refers to a structure on or in the package that is capable of repetitive fastening and unfastening (sealing or unsealing, or opening and closing) without destruction of package materials during the fastening or unfastening steps (or sealing and unsealing steps). Such resealable features may be formed integrally with planar substrates/sheets of a bag, such as during melt formation, or alternatively added later to such sheets, such as through adhesive bonding. The repetitive adherence and separation of pressure sensitive adhesive strips to package components, the interlocking and unlocking of lockable components, and the zipping or unzipping of sealing systems each constitute contemplated resealable features within the meaning of the term in this application. Additionally, it is also contemplated that more than one pair of resealable features may be used in conjunction with the bag of the invention, such as for example, a series of adjacent interlocking groove and rib structures. Interlocking groove and rib technology (and methods of making the same) can be seen in United States Patent Nos. 5,647,100 to Porchia et al., 7,137,736 to Pawloski et al., 7,410,298 to Pawloski, and 7,494,333 to Pawloski. For the purposes of this application, it should be recognized that polymeric film/sheet substrates, polymeric nonwoven sheet substrates, laminates of such, metallic foil sheet substrates, as well as woven sheet substrates are contemplated as potential bag-forming materials for the purposes of this disclosure.

Figure 1 is a front view of one suitable example of a tamper resistant and resealable bag-type package, indicated generally at 10, having easy opening features. In specific reference to Figure 1, the illustrated bag 10 has a generally longitudinal direction Y, a generally transverse direction X and a generally depth direction Z. The bag 10 can be manufactured from any suitable material, such as, for example, polymeric films and/or nonwoven sheet materials, metallic foils, woven materials or combinations of such (such as laminates of the foregoing materials). Such polymeric films and nonwoven sheets may be made for example, of polyolefinic materials as are known in the art. Such film materials may include monolayer or multilayered films or combinations of such. Such nonwoven sheet materials may include a variety of melt extruded fibrous webs (such as meltblown or spunbond materials for example), or laminate combinations of them. Such bag construction materials are desirably planar sheet-like, substrates that are capable of being stored on rolls prior to bag manufacture, or alternatively, capable of being produced in-line, such as through molding or melt extrusion processes.

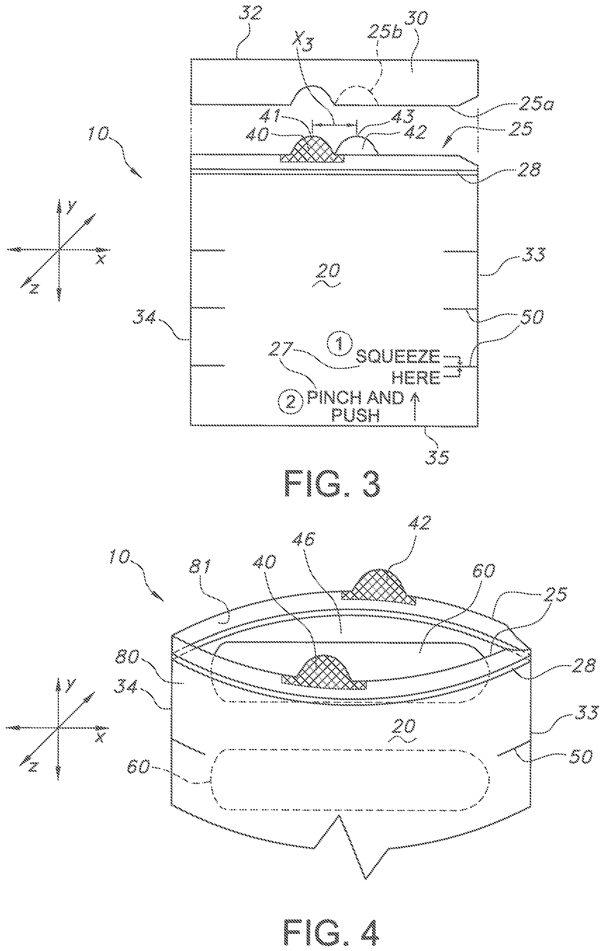

In the example illustrated in Figure 1, the bag 10 includes two distinct portions. The first portion is a bag holding portion 20 for containing consumer goods or articles 60, and the second portion is a tamper resistant strip portion 30, which may be a tear-away or otherwise removable piece of packaging (also referred to herein as a removable strip portion). The bag holding portion 20 includes a front wall 80 and a back wall 81 that cooperatively define at least part of the bag interior volume space for storing the goods or articles 60 and a bag opening 46 that can be selectively sealed by a resealable feature 28 (Figure 4). The front and back walls 80, 81 are formed from substantially planar panels that are sealingly connected along at least a pair of sides. The removable strip portion 30 is entirely or partially removed by a consumer prior to opening the bag (i.e. prior to separating/unsealing the resealable feature 28), and prior to removing the one or more of the articles 60 for use. Such removable strip portion 30 is removed, ideally, as a single piece of waste in a single removal step along a pre-defined seam and is disposed of by the consumer. The bag holding portion 20 and removable strip portion 30 are generally separated by a separating seam 25 that separately traverses each of the front and back walls 80, 81 of the bag along the bag transverse direction X from one lateral side edge to the other. It should be noted that the separating seam 25 can alternatively traverse the longitudinal direction Y (adjacent and parallel to a resealable feature 28), but such is not shown in the various figures.

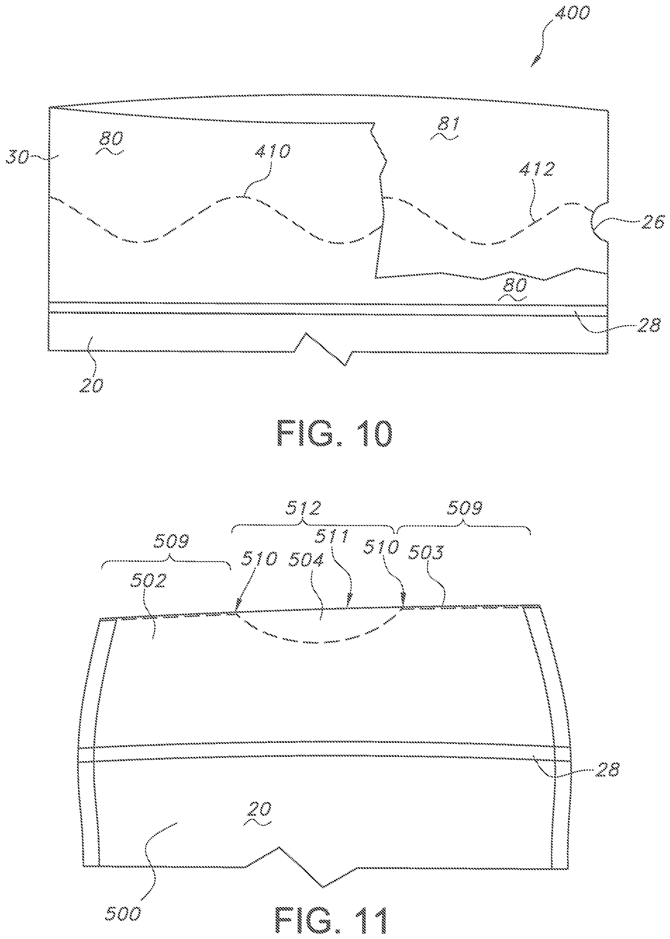

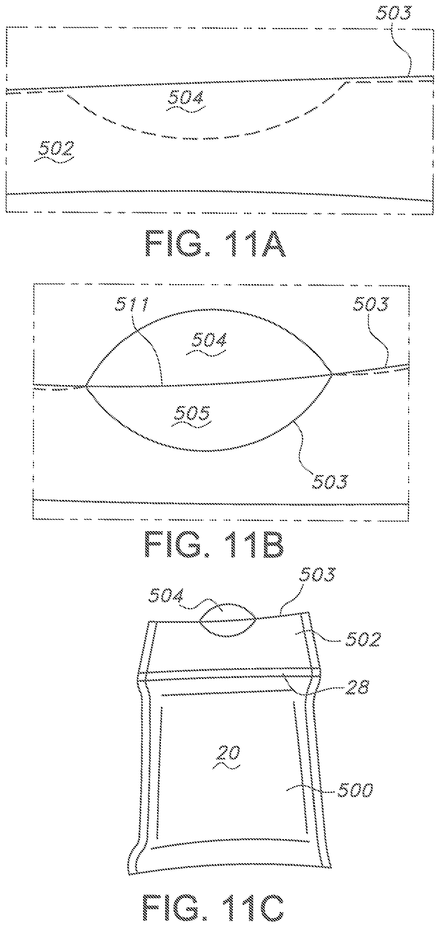

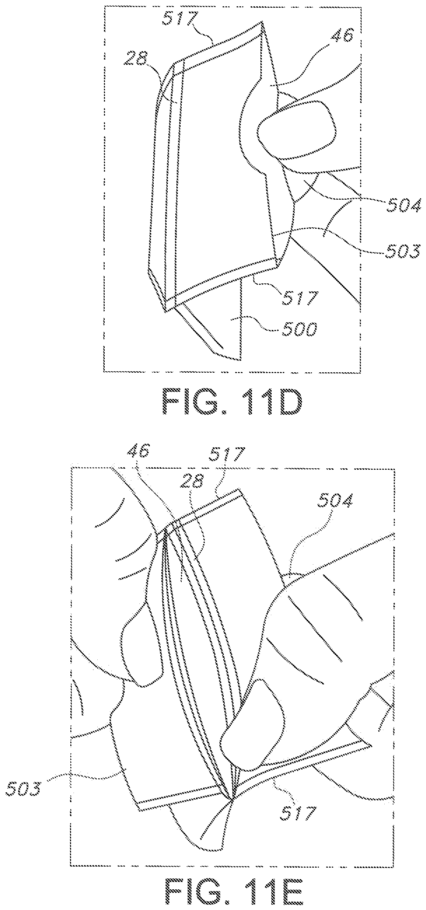

When removed, the removable strip portion 30 leaves at least one easy opening tab (a pair of opening tabs 40, 42 being seen in Figure 4) behind for opening of the resealable features 28 in the bag. In other examples and as will be described later, the tamper resistant strip portion 30 may include only a partially removable or separable tab portion (such as in Figure 11). In such an example, the tamper resistant strip portion is not itself removable, but is capable of being opened along an opening seam (503 of Figure 11) by the use of a tab (504), prior to unsealing the resealable feature 28. The structural arrangement of the bag 10 allows for the removal or opening of a tamper resistant strip portion 30, without the destruction of the easy opening tabs 40, 42 in the process. The tabs 40, 42 can be used to repetitively open the resealable feature 28 each time a user desires to remove one or more of the articles 60 from the interior of bag holding portion 20. In the illustrated example, each of the tabs 40, 42 has a generally convex configuration. In other suitable examples, however, one or more of the tabs 40, 42 can have any suitable configuration including a generally concave configuration (e.g., a cutout).

In the resealable packaging bag of Figure 1, the separating seam 25 of the package comprises two individual separating seams 25a, 25b (as seen in Figure 3). The individual separating seams 25a, 25b define the edges of the tamper resistant strip portion 30 closest to the bag bottom peripheral edge 35 of the bag holding portion 20. One of the individual separating seam 25a is on the front wall 80 and the other individual separating seam 25b is on the back wall 81. On each of the respective front and back walls 80, 81, each individual separating seam 25a, 25b defines at least one easy opening tab 40, 42. In one embodiment, the tabs 40, 42 are integral extensions of the front and back walls 80, 81 that extend into the tamper resistant strip portion 30.

In one suitable example, the tabs 40, 42 are not aligned in the Z direction, when viewed across the transverse direction of the bag 10 (as seen in Figure 3), even though straight portions of the separating seams 25a, 25b are aligned. While shown as a pair of tabs 40, 42, it is conceived that in other suitable examples only one tab may be present on the bag 10, on either the front or back wall, for assisting in opening the resealable feature 28 which is longitudinally-adjacent to the tab along the longitudinal direction Y. In another suitable example, numerous non-aligned tabs 40, 42 may be present on each bag wall 80, 81. The periphery of the bag 10 includes four peripheral outer edges 32, 33, 34, 35, visible from the front view shown in Figure 1. The front and back walls 80, 81 are sealed about their peripheral outer edges 32, 33, 34, 35 to form the bag peripheral outer edges in the embodiment of Figure 1. While a pair of individual separating seams 25a, 25b is shown in Figure 1 and immediately related figures, alternative examples can have any number of separating seams 25 defining an edge of the tamper resistant strip portion 30. For example, in Figure 11, a separating seam 503 defines the edge of the tamper resistant strip portion 502 at the upper edge of the bag 500.

In the illustrated example, the front and back walls 80, 81 are sealed to each other along their peripheral outer edges 32, 33, 34, 35 so that there is no opening in the bag 10 when it is presented to the consumer. Each of the peripheral outer edges 32, 33, 34, 35 either can be sealed by adhesive, meltbonding, or other bonding means during initial bag formation or in a post-formation process. The sealing of the bag 10 edges may occur at various production stages in various sequences. For example, the articles 60 may be loaded in the bag and then one or more of the walls 80, 81 sealed along their peripheral outer edges 32, 33, 34, 35, or alternatively, all edges may be sealed and then at least one later reopened for product loading. Specifically, the bag 10 may be loaded with articles 60 from the top peripheral edge 32 and then sealed in various locations, or loaded from the bottom peripheral edge 35 after the other peripheral edges are sealed (and the resealable feature 28 sealed). Alternatively, at least the longitudinally directed side edges 33, 34 may be sealed initially and at least one of the remaining edges 32, 35 sealed later. The resealable feature 28 may be sealed before or after all peripheral edges 32, 33, 34, 35 are sealed. In one suitable embodiment, the bag 10 may include one sheet of material that is folded to obtain two front and back walls 80, 81, such that only three side edges 32, 33, 34 (the lateral side edges along the transverse direction and top side edge) need to be bonded.

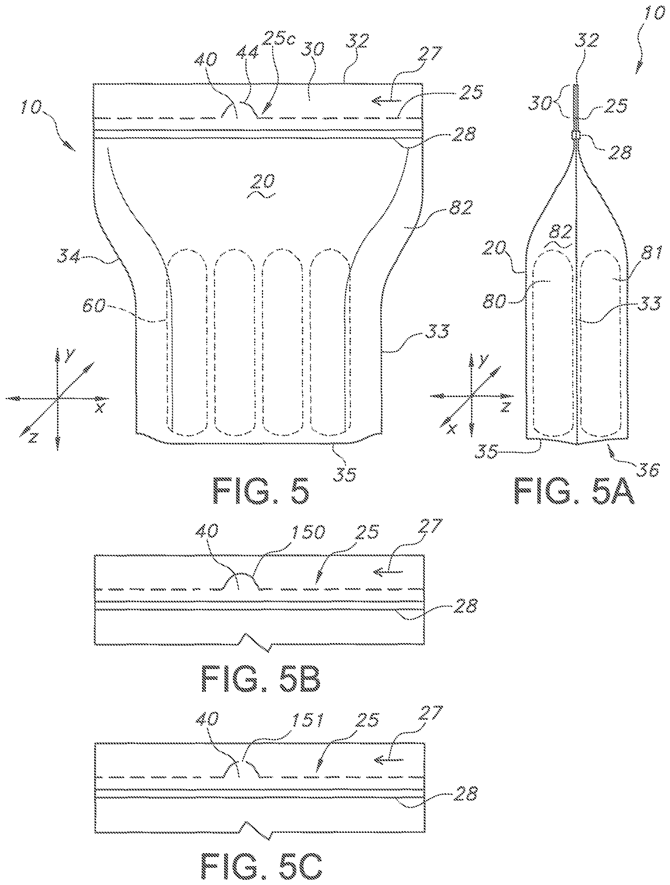

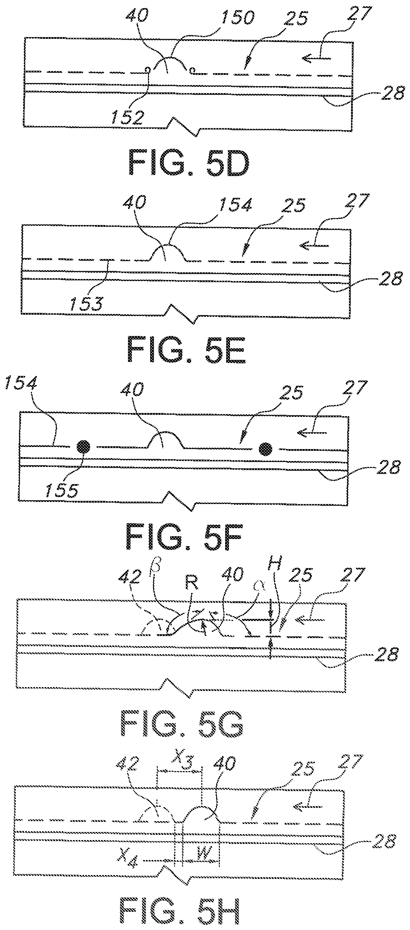

The bag 10 may be relatively flat, such as a configuration similar to a lunch-type sandwich bag, or alternatively, it may have considerable depth in the Z direction. The depth desired is dependent on the type or types of articles 60 to be stored within the bag, the flexibility of the bag construction material, and the flexibility and depth of the articles to be stored. The walls 80, 81 may be of such dimensions, or demonstrate expandable attributes, to accommodate relatively deep articles 60 (or thin articles) either packed in a single stack, or in multiple stacks within the bag 10. As seen in Figure 1, for example, a stack of four individually wrapped digital tampons are shown in phantom. Such individual tampons each have a transverse dimension X5 along the transverse direction X. In one example, the transverse dimension X5 of the each of the tampons is smaller than the transverse dimension of the bag X6 along the bottom peripheral edge 35, and the transverse dimension of the bag X7 along the top peripheral edge 32, such that the tampons may be easily inserted into and/or removed from the bag 10. In actual storage, such tampons would likely be either loaded sitting one atop the other, along the bag longitudinal direction, and parallel with the top peripheral edge 32 (and resealable feature 28) as seen in Figure 1, or in a longitudinal directed stack as seen in Figure 5.

Depending on the dimensions of the bag 10 and its ability to expand/bend with the placement of articles 60 within its walls, the expandable and/or flexible bag may form a bottom oriented side wall and lateral side walls, to accommodate the depth of the articles contained therein. For example, a two wall bag 10 is shown in Figures 5, 6, and 8, which, because of the depth of the stack of side-by-side digital tampons contained therein, has essentially created lateral side walls 82 and/or bottom wall 36, with the flexible bag wrapping about the tampons. In the example illustrated in Figure 5, the bag 10 is formed around eight articles 60 (e.g., digital tampons). As seen in Figures 5 and 5A, the articles 60 within the illustrated bag 10 are aligned four across and two deep for a total of eight articles. It is understood, however, that any suitable number of articles can be placed within the bag 10.

With reference still to Figures 5 and 5A, the holding portion 20 of the bag 10 and specifically part of the holding portion 20 spaced from the strip portion 30 generally conforms to the articles 60 contained therein to form the lateral side walls 82, which define the depth dimension Z of the bag 10, and the bottom wall 36. As seen in Figure 5A, the depth dimension Z of the holding portion 20 of the bag 10 containing the articles 60 is significantly greater than the depth dimension of the strip portion 30 and part of the holding portion 20 adjacent the strip portion. In fact, in this example, the strip portion 30 and the part of the holding portion 20 adjacent the strip portion lie generally flat (similar to a lunch-type sandwich bag), which facilities the consumer's ability to tear the strip portion 30 from the bag 10 along the separating seam 25 and the use of the resealable feature to selectively seal and unseal the bag 10 once the strip portion is removed therefrom. As a result, the bag 10 tapers inward in the depth dimension Z as viewed in Figure 5A, which is a side view of the bag. As seen in Figure 5, the part of the holding portion 20 spaced from the strip portion 30 has a smaller transverse dimension (i.e., extent in the transverse direction X) than the strip portion 30 and the part of the holding portion 20 adjacent the strip portion. As a result, the bag 10 tapers outward in the transverse dimension X as it extends upward and as illustrated in Figure 5.

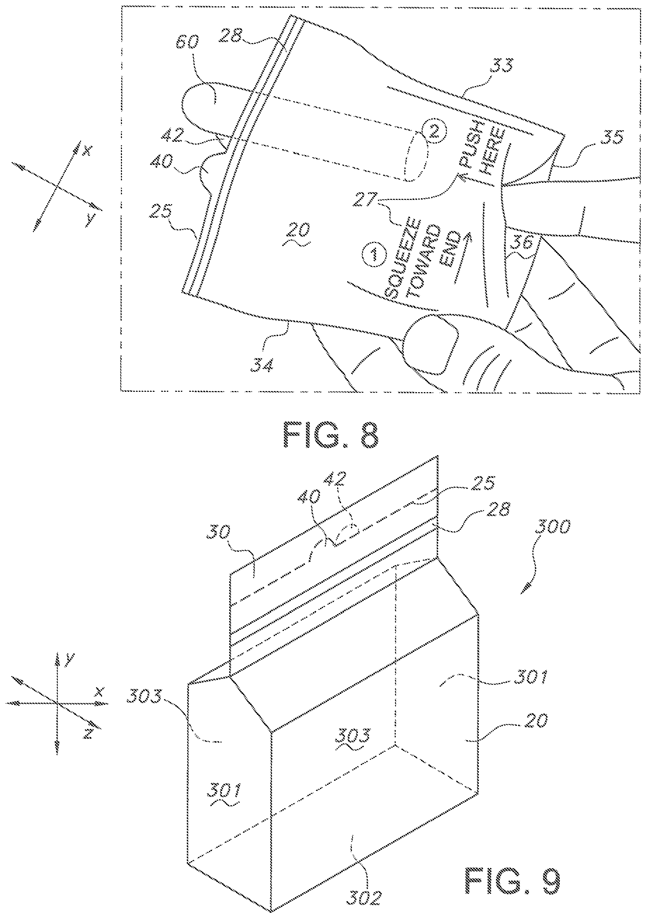

In another suitable example, as seen in Figure 9, the bag 300 may include multiple, separately formed side walls, aside from front and back walls, such as at least five walls including two relatively narrow side walls 301, a relatively narrow bottom wall 302, and a front and back wall 303 which are joined to form the bag. It is contemplated that the bag 300 may have any suitable number of walls. As with the previously described example of Figure 1, the walls of the removable strip portion 30 are sealed at the peripheral edges and a separating seam 25 and resealable feature 28 separate the removable strip portion 30 from the bag holding portion 20.

In reference again to the bag 10 of Figure 1, the illustrated resealable feature 28 is positioned below and adjacent the separating seam 25 and further separates the bag holding portion 20 from the removable strip portion 30. In this example, the resealable feature 28 is positioned between the separating seam 25 and the bag bottom peripheral edge 35 along the longitudinal direction Y. Desirably for this example, the resealable feature 28 is within about 1 and 50 mm from the separating seam 25 (shown as separating distance Y1 in Figure 1) along the longitudinal direction Y, more specifically, of the bag 10. In another suitable example, the resealable feature 28 is within about 2 and 10 mm from the separating seam 25. The resealable feature 28 may be any type of resealable feature known in the art, such as for example, interlocking groove and rib structures, zipper-like features, pressure sensitive adhesive strips, hook and loop fastener patches, magnetic closures, or combinations of multiple resealable features. An interlocking groove and rib structure is illustrated as the resealable feature 28 of Figure 1. It is desirable for the resealable feature 28 to be sealed when the consumer first receives the bag 10 such that upon initial removal of the removable strip portion 30 by a consumer (or opening of the tamper resistant strip portion), the bag holding portion 20 remains closed/sealed.

As seen in Figures 3 and 4, one of the separating seams 25a defines a tab 40 on the front wall 80, which is not centered with the tab 42 defined on the back wall 81 by seam 25b. In other respects (such as along much of their straight edge portions where there are no tabs defined), the individual separating seams 25a, 25b on each of the front and back walls 80, 81 are aligned with each other along the rest of the transverse direction X of the bag 10. The distance between the separating seam 25 and the top peripheral bag edge 32 is illustrated as Y2 in Figure 1. In one suitable embodiment, the distance Y2 is desirably larger than the distance Y1 between the resealable feature 28 and the separating seam 25.

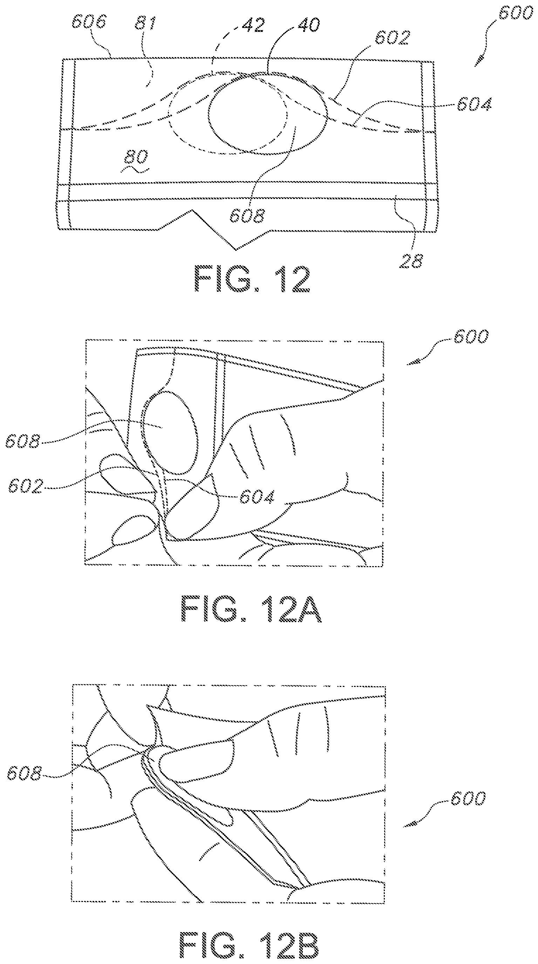

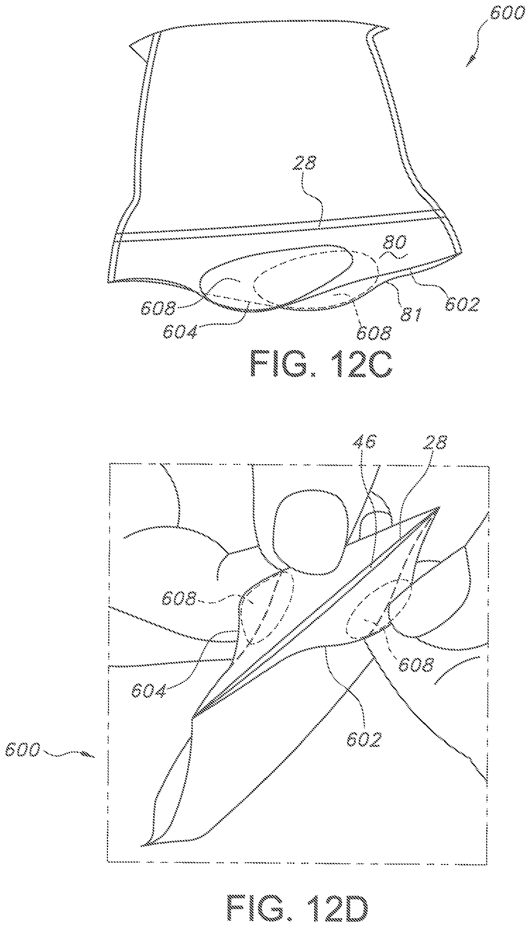

While two nonaligned tabs 40, 42 are illustrated in Figures 1-4, it should be understood that at least one tab may be present on the bag 10. For example, such tabs can number greater than two, such as three or more tabs along the bag transverse direction. Either an even or odd number of tabs 40, 42 may be present on the bag, with the number of tabs on respective and opposed front and back walls 80, 81 either being equal to each other or not. Desirably, at least one tab 40, 42 is present on each of the front and back walls 80, 81. In examples having other than two walls, the tabs 40, 42 can be present on any number of walls. The defined tabs 40, 42 can have any number of different shape profiles, although semicircles are illustrated. Tab profile shapes include, for example, rectangular, triangular, curvilinear, asymmetrical shaped, or a combination of such. The tab shape profiles on each of the front and back walls 80, 81 may be the same or different from one another on each wall, or between walls. The tab profiles can extend along much of the bag transverse direction, or very little distance along the bag transverse direction. In this regard, such numerous tabs or extended tab profiles are seen in Figures 10, and 12-12D. As mentioned above, one or more of the tabs 40, 42 can have a generally concave configuration (e.g., a cutout) without departing from some aspects of this disclosure.

The degree by which the tabs 40, 42 are not aligned (or not centered) between the two walls 80, 81 can vary. For example, the tabs 40, 42 can be 100% out of alignment between the two walls 80, 81, (in that there is no surface area overlap in the Z direction between the tabs on opposed walls), or alternatively, less than or equal to 90% out of alignment (having some surface area overlap in the Z direction). Desirably, there is an alignment less than or equal to about 50%, in that less than 50% of the opposing tab surface areas are overlapping in the Z direction. In other suitable examples, involving multiple tabs on each of the front and back walls (not illustrated) some of the tabs may be in alignment between the front and back walls, while others may be out of alignment in the Z direction.

Desirably, in one example, the tabs 40, 42 are semicircular in profile shape and include a distance X3 between neighboring peaks 41, 43 along the transverse direction X3, of between about 1 and 75 mm, more desirably, between about 5 and 30 mm, and even more desirably, between about 5 and 20 mm (Figure 3). The ranges of distance X3 noted above apply to both the distance between immediately adjacent peaks 41, 43 on the same wall of a bag, or between adjacent, opposing peaks of two distinct walls (as illustrated). It should also be noted that in one suitable example, as seen in Figure 3, the heights H of neighboring peaks 41, 43 are the same. In other suitable examples, the heights of neighboring peaks 41, 43 are different. In other suitable examples, the maximum heights of peaks 41, 43 vary across the same wall of a bag. In yet other suitable examples, the peaks 41, 43 of tabs 40, 42 are not centered within the tabs (such that tabs are of asymmetrical shapes). If the adjacent tabs 40, 42 are farther apart along the transverse direction X (either along the same wall, or between opposing walls), it presents a greater challenge for a consumer to visually and tactilely view the tabs as a pair for use together in opening the bag. See the distance measurement X4 in Figure 5H for example.

With reference to Figures 5G and 5H, the height H (or amplitude), width W (or frequency), radius R, approach angle a, and retreat angle β will depend on the size of the bag 10 and the types of materials employed in constructing the bag (e.g., the tabs 40, 42 and the walls 80, 81). More specifically and with respect to the size of the bag 10, the height H, width W, radius R, approach angle a, and retreat angle β will depend on the distance between the top peripheral bag edge 32 and the resealable feature 28 (i.e., the sum of the distance Y1 and the distance Y2 as seen in Figure 1). In connection with Figure 5G, it is desirable, in one suitable example, for the height H of the tab peaks to be between about 1 and 50 mm, from their base at the separating seam 25 to their highest point. In other suitable examples, the height H can be between about 5 and 50 mm and more specifically between about 7 and 15 mm. In one suitable example, for example, the height H of the tab peak can be about 1.3 mm. In another suitable exemplary example, the height H of the tab peak can be about 3.2 mm.

As can be seen in Figure 5H, the tabs in one example have a width W at their base along the transverse direction, from where the elevated portion of the tab begins to project from the separating seam 25. It is desirable, in one example, for the base width W of the tabs 40, 42 to be between about 5 and 75 mm, and more desirably to be between about 5 and 30 mm. In one suitable example, for example, the base width W of the tabs 40, 42 is about 11 mm. In another suitable example, the base width of the tabs 40, 42 is about 16 mm. If the width W is too narrow, the tab 40, 42 may be more susceptible to being torn off with the removable strip portion 30. Desirably, the distance X4 between the bases of adjacent tabs 40, 42 along the separating seam is between about 0 and 50 mm, more desirably, between about 0 and 20 mm, and even more desirably, between an amount greater than 0 and less than about 20 mm. This distance X4 required to prevent tear off depends on the overall size and construction materials of the bag 10. For distances of about 0, such tabs between opposing walls may demonstrate some surface area overlap in the Z direction.

As illustrated in Figure 5G, the approach angle α is defined at the location where the tab 40, 42 begins to project from a generally linear portion of the separating seam 25, and the retreat angle β is defined at the location where the tab 40, 42 transitions back to the generally linear portion of the separating seam 25. In one suitable example, the approach angle α is between about 110 and 160 degrees and, more preferably, between about 120 and 150 degrees. In one suitable example, for example, the approach angle α is about 135 degrees. In other suitable examples, the approach angle α can be about 132 degrees or about 149 degrees. It is contemplated that the retreat angle β can be equal to or different than (i.e., greater than or less than) the approach angle a. For example, as illustrated in Figure 5G, the retreat angle β can be different than the approach angle a.

With reference again to Figure 5G, in one suitable example, each of the tabs 40, 42 has a radius between about 5 and 15 mm. In one example, each of the tabs 40, 42 has a radius of about 10 mm. It is contemplated that the radius R of the tabs 40, 42 can be uniform (i.e., the same throughout the extent of the tab) or can vary along the tab's profile. In one suitable embodiment, the tabs have a first radius adjacent the approach angle α and a second radius adjacent the retreat angle β (Figure 5G) that is different than the first radius. In one embodiment, for example, the first radius is less than the second radius. Alternatively, the second radius can be less than the first radius.

In one embodiment, the height H is substantially equal to the radius R of the tab 40, 42 at about at least a portion of the tab's profile. In one suitable example, the height H is substantially equal to the radius R of the tab 40, 42 at a location adjacent at least one of the approach angle α and the retreat angle β. For example, both the height H and the radius of the tab 40, 42 at a location adjacent at least one of the approach angle α and the retreat angle β can be approximately 3.2 mm. In another suitable example, the height H is less than the radius R of the tab 40, 42 about at least a portion of its profile. In one such example, the height is less than the radius R of the tab 40, 42 at a location adjacent at least one of the approach angle α and the retreat angle β. In one example, the radius R of the tab 40, 42 at a location adjacent at least one of the approach angle α and the retreat angle β is at least twice the height H (i.e., if the tab 40, 42 has a height H of 3.2 mm then the radius would be 6.4 mm or greater). In another example, the radius R of the tab 40, 42 at a location adjacent at least one of the approach angle α and the retreat angle β is at least five times the height H (i.e., if the tab 40, 42 has a height H of 3.2 mm then the radius would be 16 mm or greater). In yet another example, the radius R of the tab 40, 42 at a location adjacent at least one of the approach angle α and the retreat angle β is at least ten times the height H (i.e., if the tab 40, 42 has a height H of 3.2 mm then the radius would be 32 mm or greater). In yet another suitable example, the height H can be greater than the radius R of the tab 40, 42 about at least a portion of its profile. In one such example, the height is greater than the radius R of the tab 40, 42 at a location adjacent at least one of the approach angle α and the retreat angle β.

It is contemplated that the tabs 40, 42 of the bag 10 can have any suitable height H, width W, radius R, and/or approach angle α without departing from some aspects of this disclosure. Preferably, each of the height H, width W (or frequency), radius R, approach angle a, and retreat angle β are selected based, at least in part, on the size and material of the bag 10 to inhibit the tabs 40, 42 from being torn off with the removable strip portion 30.

In some suitable examples, the tabs 40, 42 of the front and back walls 80, 81, can be of different colors or textures from one another, so as to help facilitate easy opening (visually or tactilely) of the bag once the removable strip portion 30 has been removed from the bag holding portion 20. Such color or texture can be formed as an integral part of the walls 80, 81 from which the tabs 40, 42 extend, or alternatively, such color or texture differences can be the result of a limited applied color or texture, such as by the application of a printed design, coating, or application of an adhesive decal solely to the tab surface area (and not the entire wall from which the tab extends). In yet another suitable example, the entire front and back walls 80, 81 may be of different colors or textures from one another so as to facilitate easy visualization and opening. In still a further alternative example, the differences in color or texture on the tabs may be the result of a color or texture difference being applied only to the outwardly directed user facing surfaces of the tabs 40, 42 (or the inwardly facing surfaces). The outwardly directed user facing surfaces of the tabs 40, 42 are those surfaces which do not face the opposing bag walls 80, 81.

The separating seam 25, and in particular, the individual separating seams 25a, 25b of each of the front and back walls 80, 81 respectively, may include perforations or otherwise weakened areas, embossments, or indentations, so as to facilitate ripping, tearing, or opening of the tamper resistant strip portion 30. Such perforating or weakening may for example be accomplished by a targeted laser scribing technique but it is understood that any suitable laser technique can be used. The seam of perforations or otherwise weakened portions 90 can include a series of dashes (i.e., dash-shaped perforations) along the bag 10 transverse direction (as shown in Figure 1). Such dash-shaped perforations or weakened portions 90 can be any number of individual lengths X1 along the transverse direction. Desirably, in one suitable embodiment, each individual length X1 is between about 0.25 and 10 mm, and more desirably, between about 0.5 and 3 mm. Dash-shaped perforations have a length dimension longer than the width dimension of the perforation. Essentially, the length dimension along the X direction is larger than the width dimension along the Y direction. Such dashes can be uniformly dimensioned (uniform lengths) across the transverse direction, or of different lengths. Such perforations or weakened areas 90 may consist entirely of dot-shapes (not shown in Figure 1) rather than dash-shaped perforations (as shown in Figure 1), or a combination of the two (as seen in Figure 5F). In one suitable example, a distance of separation X2 between adjacent perforations or weakened areas 90 along the transverse direction (X2) is between about 0.25 and 5 mm, for dash shaped perforations, and more desirably, between about 0.5 and 1 mm (as seen in Figure 1). For dot-shaped perforations (perforations in which the length and width dimensions are approximately equal) in one embodiment, the distances of separation X2 between adjacent dots is desirably between about 0.1 and 2 mm. The perforations or weakened areas 90 may be uniformly spaced across the bag transverse direction X or non-uniformly spaced. In one suitable example, the individual perforations or weakened areas intersect the peripheral lateral bag edges 33, 34 to facilitate easy tearing(as seen in Figure 1), rather than terminating inside the peripheral edges. In another suitable embodiment, the individual perforations or weakened areas 90 that make up the separating seam 25 are perpendicular to the bag peripheral edges 33, 34 at least at locations immediately adjacent the bag lateral peripheral edges 33, 34.

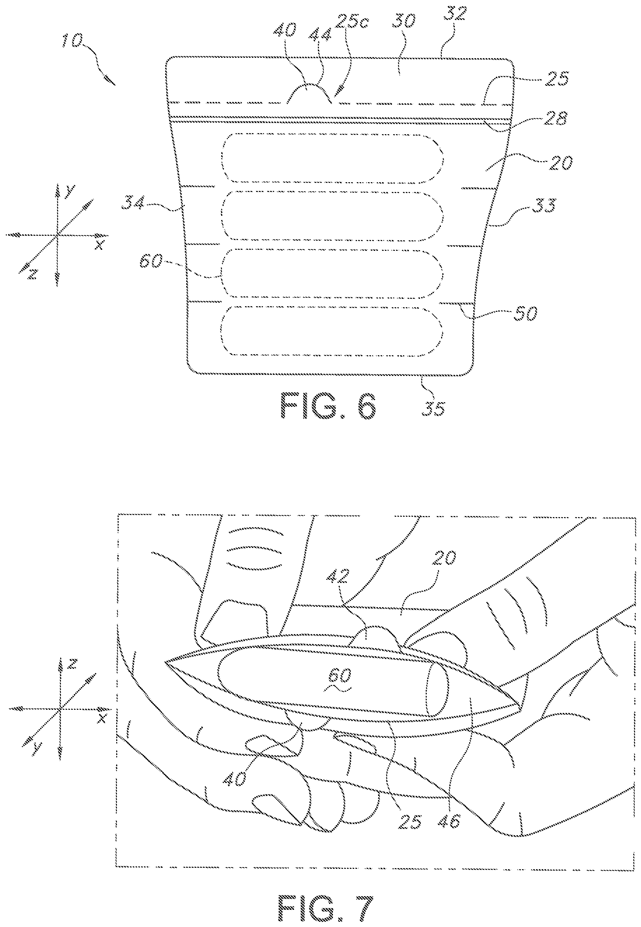

Various perforation or weakened area patterns are contemplated for facilitation of removal or opening of the tamper resistant strip portion 30 along the separating seam 25. For example, in one example, such perforation or weakened areas 90 are extended over only a portion of the periphery of the tab 40. Such an extended perforation 44 can start at the straight seam areas 25c and extend onto the defined tab areas (as seen in Figure 5). In another suitable example, the extended perforation 44 can traverse the tab periphery from one side to the other (as seen in Figure 6). As seen in Figure 5B, such extended perforation 150 can start from a straight portion adjacent and aligned with the separating seam 25 on one side of the tab 40, traverse the periphery of the tab, and continue to another straight portion on the other side of the tab. As can be seen in Figure 5C, such perforation or weakened area defining the tab 40, can be split into at least two extended areas 151. As can be seen in Figure 5D, the perforated or weakened areas 90 on the straight portions of a seam can have corner portions 152 that are directed away from the tab 40 to prevent inadvertent ripping off of the tab. The corner portions 152 are curved away from the tab. As can be seen in Figure 5E, the perforated or weakened areas can include extended areas 153 on a straight portion of a seam and shorter areas 154 about the tabs 40, 42.

As a result of the propensity of some consumers to tear off removable strip portions from tamper resistant packages in a quick or jerky manner, in some examples of the described bag 10, it may be useful to provide either a reinforcement material or zoned strength element to the offset tabs 40, 42, or alternatively to provide further tear enhancement features to the separating seam 25 as previously noted. For example, in one example, the tabs 40, 42 and a region 47 extending from the bottom of the tabs to beyond the separation seam 25 in the Y direction have a basis weight larger than much of the adjacent walls 80, 81 forming bag holding portion 20. In another example, a reinforcing coating or treatment extends over the tabs 40, 42 towards the bag bottom end (in the Y direction) in a particular region 47. In a further alternative embodiment, each tab 40, 42 is strengthened by a reinforcing decal or other applique placed on the tab and a portion of the wall beneath the separating seam along the Y direction. Such reinforcement material or zoned strength element may be added to either side or both sides of the tabs 40, 42. In yet another example, the perforation 25c defining the tabs 40, 42 extends from a seam straight portion over a larger peripheral edge of the tab 40, such as over 25 %, desirably, over 50 %, and even more desirably, over 100 % of the tab peripheral edge (as seen in Figures 5, 5B, 5C, 5F). Desirably, the continuous perforation line around the tab peripheral edge starts on the side of the tab 40 facing the tear initiation point such as a rip/tear starting feature 26. In this fashion, a tear/rip will more easily be propagated around the tab, without ripping the tab off in the process of removing removable strip portion 30.

In some examples, the separating seam 25 may further include a rip/tear starting feature 26, such as a perforation adjacent and extending to the side edge 33 or a cut-out carrot, to allow for easy tear initiation for the removal of the removable strip portion 30 along the separating seam 25. The cut-out carrot may be a V-shaped, U-shaped, semi-circular shaped, or any other suitable cut-out feature. Such rip/tear starting feature 26 also serves as a visual indicator to illustrate where the tear should be initiated by the consumer. The bag tamper resistant strip portion 30 and/or bag holding portion 20 may optionally include guiding instructions in the forms of words or symbols (such as an arrow) to assist the consumer in opening the bag or removing the strip portion.

To facilitate dispensing of a single article 60 from the bag 10, guide words or symbolic indicators 27 can be placed on the bag, e.g., along the bag length 50 and/or at one end of the bag, such as adjacent the bag bottom edge 35. The placement of such indicators 27 will depend on the desired orientation of the articles 60 in the bag 10. For example, as can be seen in Figures 1, 4, and 6, the articles 60 (in this case digital tampons) are stored in a horizontal configuration parallel to the bag opening 46 aligned along the bag transverse direction X. It should be noted that while digital tampons are illustrated in the figures, such a bag can be used to store and dispense any number of articles, and in particular personal care articles, such as for example applicator tampons and rolled panty liners (as are known in the art). A series of dispensing steps can be visually emphasized on the bag to assist the consumer in dispensing individual articles 60 from the package. For example, as seen in Figure 1, the consumer can "1" squeeze the bag at a particular point 50 and then "2" pinch and push the article upward as desired.

As can be seen in Figure 2, a tear has been initiated along the separating seam 25 to begin separating the removable strip portion 30 from the bag holding portion 20. As can be seen in Figure 3, the removable strip portion 30 has been completely separated from the bag holding portion 20, exposing the offset, non-centered, or staggered tabs 40, 42. In one suitable example, the separating seam 25 has a tear strength less than the tear strength of the bag 10. As can be readily appreciated, the bag 10 has a tear strength in both the longitudinal direction Y and the transverse direction X. The tear strength of the bag 10 in the longitudinal direction Y can be equal to, greater than, or less than the tear strength of the bag in the transverse direction X. Suitably, the tear strength of the separating seam 25 is sufficiently less than the tear strength of the bag 10 in both the longitudinal direction Y and the transverse direction X to inhibit other portions of the bag (e.g., tabs 40, 42) from being torn during removal of the removable strip portion 30. In one suitable example, the tear strength of the separating seam 25 is less than the lesser of the bag's tear strength in the longitudinal direction Y or the transverse direction X by at least 1%, and suitably by at least 5%, and more suitably by about 25%. The tear strength of the separating seam 25, however, should be sufficient to protect the integrity of the bag 10 until the consumer desires to purposefully tear the removable strip portion 30 from the bag 10 along the separating seam 25. Suitable methods for testing tear strength include, but are not limited to, ASTM D1424-96 Standard Test Method for Tearing Strength of Fabrics by Falling-Pendulum Type (Elmendorf) Apparatus and ASTM D 1922, Standard Test Method for Propagation Tear Resistance of Plastic Film and Thin Sheeting by Pendulum Method. It is contemplated that other suitable testing methods could be used.

In one suitable example, the tear strength of the separating seam 25 can vary along its length. In one such example, the separating seam 25 is weakest (i.e., its lowest tear strength) at or adjacent to the portion of the separating seam that projects from the linear portion to define the tab 40, 42. In other words, the portions of the separating seam 25 that define either the approach angle α or the retreat angle β are the weakest portions of the separating seam 25 to inhibit the tabs 40, 42 from being torn during removal of the removable strip portion. Relative weakening of the separation seam 25 can be accomplished by, for example, increasing the length of the cut-out portions, if perforated, or by cutting deeper in the z direction of the bag, if a line of weakness is used. In one suitable example, the portions of the separating seam 25 that define either the approach angle α or the retreat angle β are at least two times weaker than the linear portion of the separating seam. In another suitable embodiment, the portions of the separating seam 25 that define either the approach angle α or the retreat angle β are at least three times weaker than the linear portion of the separating seam. In yet another suitable example, the portions of the separating seam 25 that define either the approach angle α or the retreat angle β are at least five times weaker than the linear portion of the separating seam.

As can be seen in Figure 4, the resealable feature 28, in this case the interlocking groove and rib structures, has been separated, allowing the removal of one or more article 60, such as the illustrated individually wrapped digital tampons. Once the resealable feature 28 is opened via pulling apart the tabs 40, 42, such articles 60 are desirably dispensed from the bag 10 by squeezing the bag at one of various squeeze points 50 indicated by squeeze guide indicators 27 using the thumb and forefinger at opposite front and back walls 80, 81 of the bag. If one or more articles 60 had previously been removed through the bag opening 46, then the same squeezing technique would be applied to the bag 10, accompanied by a pinching/dragging or pushing motion of the fingers towards the bag opening. Such squeezing and pinching/dragging or pushing motion is illustrated in Figure 7. As illustrated in Figure 8, if the stored articles 60 are packaged along the length direction of the bag 10, the push guide indicators 27 can be positioned (in words or symbols) at the bag bottom wall 36 or along the front or back walls 80, 81 for guiding the consumer to dispense one article at a time. Once an article 60 is dispensed, the resealable feature can then be sealed again for dispensing of individual articles at a later time. The tabs 40, 42 can be used repetitively to assist in opening the resealable feature 28. In one suitable embodiment, the resealable feature 28 requires an operational force between about 3 and 10 Newtons to open the resealable feature. In the illustrated example, for example, the operational force of between about 3 and 10 Newtons is required to disengage the rib from the groove.

Another suitable example of the bag 400 is illustrated in Figure 10. As can be seen, the bag 400 includes a resealable feature 28 and staggered, multi-peaked seams 410, 412 on each of the front and back walls 80, 81 of the two-walled bag. The top peripheral edge of the bag may be sealed either before or after the creation of the seams 410, 412.

In yet another suitable example, as seen in Figures 11-11E, instead of including the removable strip portion 30 immediately adjacent the resealable feature 28, a bag 500 includes an openable tamper resistant strip portion 502 that is not necessarily removed. Such strip portion 502 includes two walls (as with previously described designs) that are eventually sealed at their top peripheral edge, but includes a perforation or weakening seam 503 along or adjacent the bag upper edge. The seam 503 includes straight portions 509 and a curved portion 512 separated by junctures 510. The perforation or weakening seam 503 defines at least a single foldable tab 504, which is an extension of one of the walls that folds over the other wall. As with previous examples, the tab 504 may be integral with a wall, or a non-integral attachment to a wall. A cut-out area 505 (as seen in Figure 11B) is optionally present in the other wall. As the foldable tab 504 is opened along fold line 511 (by popping the weakened or perforated areas of the tab with one's finger), the tab 504 is grabbed and lifted away from the underlying wall. The fingers are then used to pull the tab 504 away from the underlying wall along the curved portion 512 of seam 503, thereby separating the remaining perforated or weakened seams 503 such as in the straight portions 509. Such separation allows for the separation of the resealable features 28, and the eventual dispensing of an article contained within the bag through the bag opening 46. The tamper resistant strip portion 502 includes lateral peripheral side edges 517 that are sealed adjacent the resealable feature 28. In one example, such tamper resistant strip portion 502 is made from a single planar sheet that has been folded over to create the bag edge, and then perforated or weakened along the perforation or weakening seam 503. In another suitable example, two separate planar sheets are used to create the bag walls. In another suitable example of this folded tab design, the tamper resistant strip portion 502 is perforated along lateral side edges 517, to enable the tamper resistant strip portion 502 to be opened on three sides. In another embodiment, the tamper resistant strip portion 502 can be opened along the length of lateral side edges 517 all the way to the resealable feature 28, as illustrated in Figure 11E.

In still another suitable example, as seen in Figures 12-12D, a bag 600 includes a series of perforated or weakened seams 602, 604 situated between a bag sealed upper edge 606 and a resealable feature 28. The series of seams 602, 604 define at least two offset tabs 40, 42 and may be created by perforating or weakening the bag from one side only or from both sides. In some examples, especially when the seams 602, 604 are formed in both walls from one side only, both seams are on each of the walls 80, 81, i.e., both seams 602, 604 are on the front wall 80 and both seams 602, 604 are on the back wall 81, even though only one seam is intended to be opened. So as to prevent one seam from being unintentionally opened during separation of the removable tamper resistant strip portion and opening of the bag, an adhesive decal 608 is placed on the outwardly facing surfaces of the bag, or the inwardly facing surfaces, over the seams 602, 604 that are unintended to be opened. At least one adhesive decal 608 is placed on each wall 80, 81 of the bag 600 on the removable strip portion, to thereby allow the tabs 40, 42 to be separated when pulled from opposing sides once the removable strip portion has been removed along seams 602, 604. In the operation of this example, the removable strip portion above the perforated or weakened seams in the Y direction is torn free. The front wall decal 608 and front wall tab 40 remain on the front wall 80 of the bag 600. The back wall decal 608 and back wall tab 42 remain on the back wall 81 of the bag 600. The placement of the decals 608 negates the effect of the unused perforation or weakened areas on the walls (especially when the perforations or weakened areas are created in both walls from one wall side only). When viewed from the reverse tab sides, the back perforation line is visible on the front wall 80 of the bag. The front perforation line is visible on the back wall 81 of the bag. The tabs 40, 42 are each held together in part because of the adhesive decals 608. The combination of offset perforated tabs 40, 42 and decals 608 provide easy opening features with enhanced visibility, grip, and strength (as a result of the decals) for use in separating the resealable features 28 to create an opening 46.

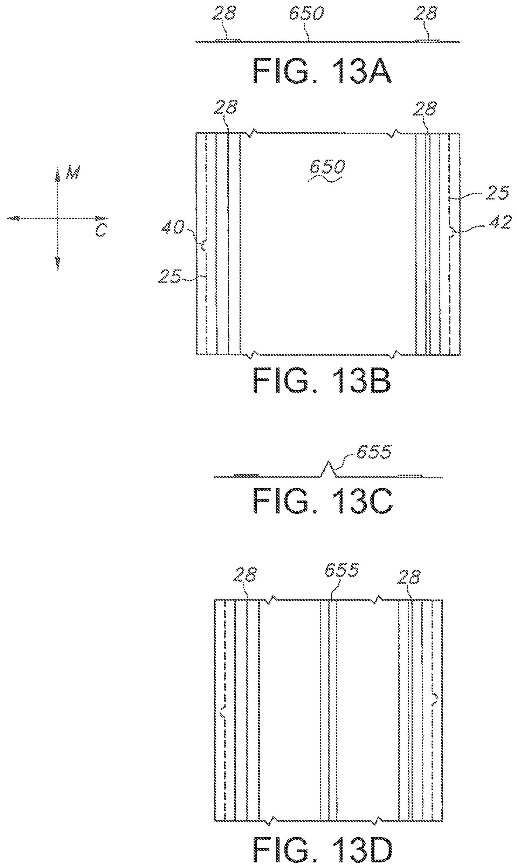

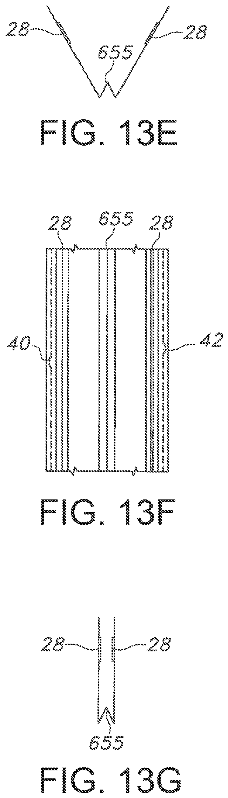

Bags with offset tabs (as described below) can be made by various production methods. For example, in one suitable production method, as illustrated in Figures 13A-13G, a planar sheet material having opposing seams and resealable features is produced along the machine direction. Figures 13B, 13D, and 13F illustrate several steps of production in the machine direction, with Figures 13A, 13C, and 13E illustrating the cross machine directional views of respective Figures 13B, 13D, and 13F. As seen in Figure 13B, generally opposed tabs 40, 42 created by perforations or weakened areas on separating seams 25, are offset from one another (not centered with respect to one another) along the machine direction. The planar material 650 includes resealable features 28 that are adhered to the sheets in the machine direction. The planar material 650 is folded at fold lines 655, such that a bag bottom is created. The planar material is optionally folded in a second step, and the walls brought together, such that the resealable features 28 are aligned (such as the groove and rib of an interlocking resealable feature 28). The straight portions of individual separating seams 25a, 25b are likewise aligned and the bag is cut in the cross-machine direction, such that opposed, noncentered tabs 40, 42 are in the cut areas. The bag is sealed at its resealable feature 28, and at its lateral and top side edges (lateral edges created in the cutting step). Articles may be loaded in the bags before sealing.

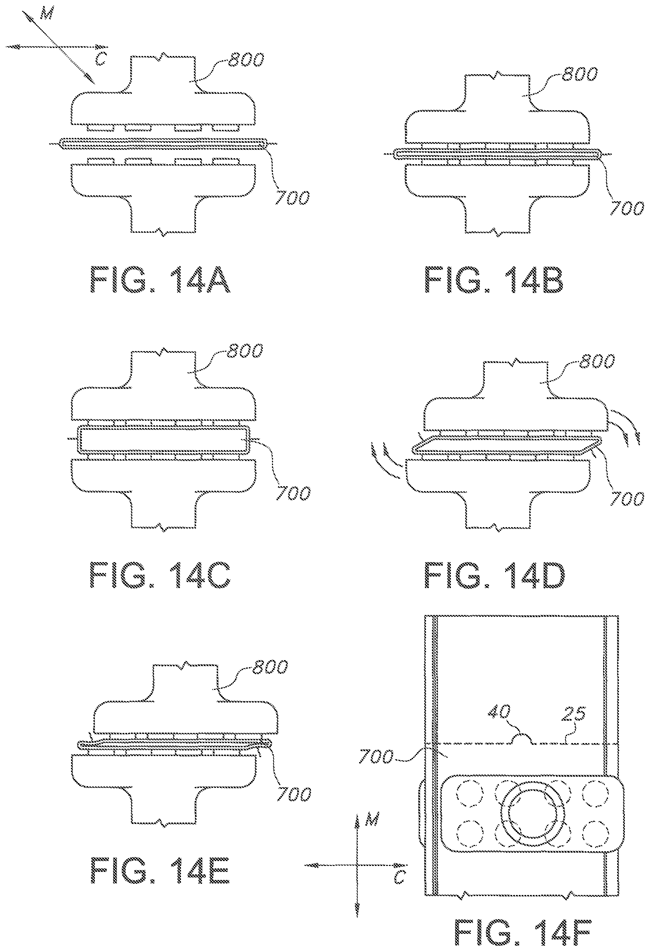

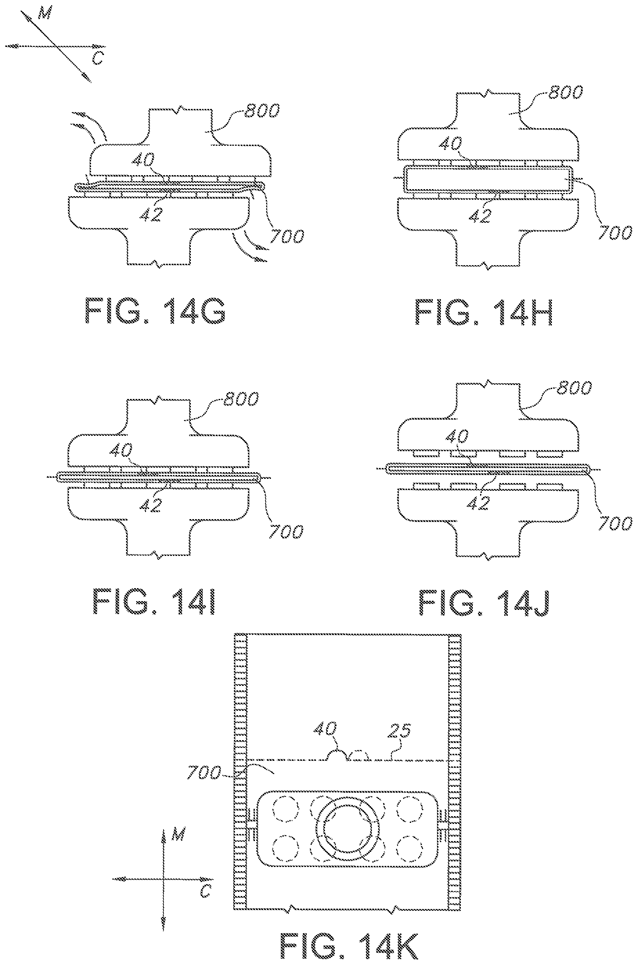

In another suitable production method, which is illustrated in Figures 14A-14K, a bag 700 is produced in the machine direction using a vacuum device 800 for offsetting tabs on a preformed bag with resealable features. The tubular bag-like structure 700 having open ends and sealed lateral edges is fed into the machine direction of a two-sided vacuum device 800, as seen in Figure 14A. The vacuum device 800 includes an upper and lower portion. The vacuum device 800 advances against the tubular bag-like structure surfaces as seen in Figure 14B. Following approach to the bag-like structure surfaces, the vacuum device retracts and pulls the bag-like structure 700 to an open position as seen in Figure 14C. During retraction of the vacuum device 800, the width of the bag-like structure 700 decreases in the cross-machine direction. Following this step, the upper and lower vacuum portions of the device advance towards each other in opposite directions such as to tilt the bag-like structure 700 on its side. This movement causes the upper and lower surfaces of the bag-like structure 700 to move in opposite directions, and the surfaces to approach each other. The distance that the vacuum device 800 shifts to each side is equal to the desired displacement of the tabs 40, 42 on the bag (the separation distance between the tabs on the bag). At this point, as seen in Figure 14F, the tilted bag-like structure 700 is perforated or otherwise weakened through both walls, from one side to create a separating seam 25 that penetrates through both wall layers and defines the tabs 40, 42. The tabs 40, 42 are fully aligned along the cross-machine direction as seen in Figure 14G. Then the vacuum device 800 either separates and moves the bag-like structure 700 or the vacuum device slides the bag-like structure sideways and returns it to its original orientation as seen in Figures 14I and J. At this point, as seen in Figure 14K, the bag-like structure 700 includes the off centered perforated tabs 40, 42 on a separating seam 25. The bag-like structure 700 may be cut and at least partially sealed as a bag. Products may be then loaded into the partially sealed bag 700. A resealable feature may be provided with the bag-like structure 700 prior to perforation, or alternatively at a later step.

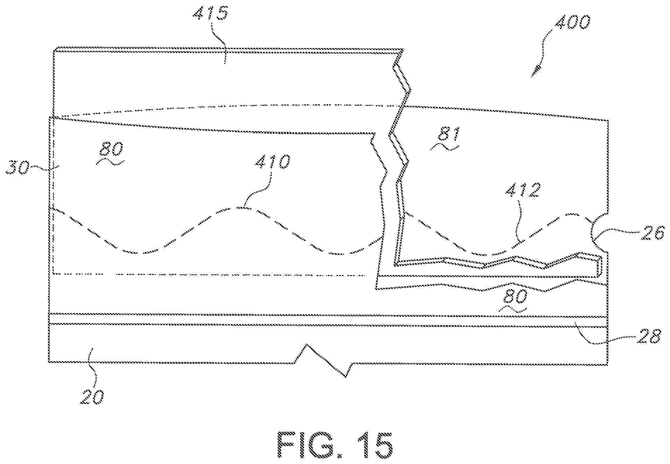

As can be seen in Figure 15, in one suitable production method, the seams of two walls of the bag 400 illustrated in Figure 10 may be perforated separately while a barrier plate 415 is used to separate the two walls 80, 81 from one another. The two separating seams 410 and 412 could have different paths on each wall to create off centered tabs along the full transverse direction of the bag. The separating seams 410, 412 join together in the Z direction at an optional tear starting feature 26, in this embodiment a semicircular cutout, that can be used to initiate tearing or ripping along the seams. The barrier plate 415 would not necessarily be needed to create perforations if a laser scribing perforation method is employed. The staggered, multi-peaked seams 410, 412 are created by inserting the barrier plate 415 within the bag, while the top peripheral edge is unsealed, and then perforating or weakening the opposite bag walls 80, 81 along seams with staggered patterns. The barrier plate 415 prevents the perforation of one wall from penetrating the opposite wall when using traditional perforation techniques. Although not illustrated, a perforation device can be placed in the position of the barrier plate to provide independent wall perforations to each opposing wall. An anvil-like device can then compress the bag to create independent perforation patterns on each bag wall, with the independent perforation patterns including noncentered tabs.

It should be noted that in several of the described methods, the order of steps in bag production can be changed. For example, the steps of the sealing of a resealing feature 28, the sealing of bag peripheral edges, the cutting of bag planar sheet materials, the loading of the articles 60, and the perforation/weakening of separating seam 25 can be moved in a method to accommodate machinery placement along a manufacturing line.

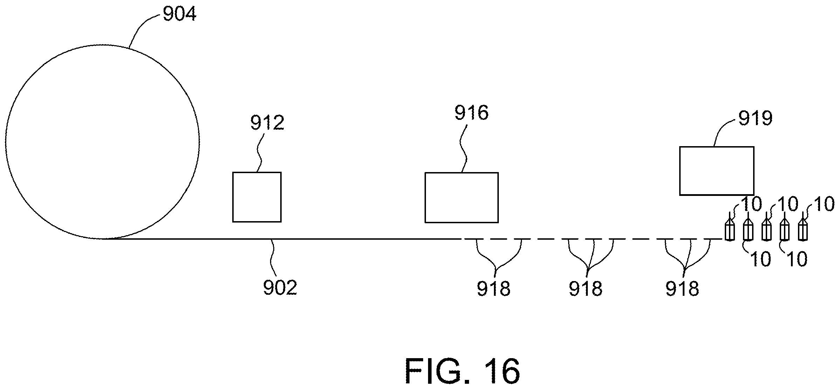

As mentioned above, the separating seam 25, and in particular, the individual separating seams 25a, 25b of each of the front and back walls 80, 81 respectively can be formed using a targeted laser scribing technique. Figures 16-18 illustrate one suitable production method for forming bags in accordance with this disclosure wherein a targeted laser scribing technique is used to form at least one of the individual separating seams 25a, 25b. For illustration purposes only, a plurality of the bag 10 of Figures 5 and 5A is illustrated being formed in Figures 16-18.

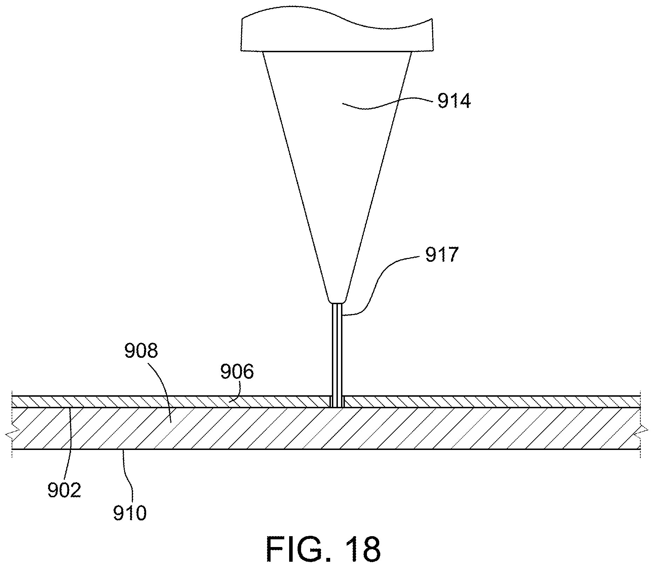

As schematically illustrated in Figure 16, a continuous web 902 suitable for the construction of a plurality of the bags 10 is fed from a suitable supply roll 904 (or other suitable source of web including the in-line manufacturing of the web). As explained above, the material of the bags 10 and thus the continuous web 902 can be any suitable material including monolayer or multilayered films. In the illustrated embodiment, the continuous web 902 is a multilayered film having a first layer 906 and a second layer 908 bonded to the first layer (Figure 18). In one suitable embodiment, the first layer 906 is adhesively bonded to the second layer 908 but it is contemplated the first and second layers can be bonded using any suitable bonding technique. In the illustrated embodiment, the first layer 906 is adapted to define the outer surface of the bag 10 and the second layer 908 is adapted to define the inner surface of the bag. More specifically, in the embodiment illustrated in Figures 16-18, the first layer 906 has a thickness of about 0.018 mm, and the second layer 908 has a thickness of about 0.062 mm. Thus, the illustrated laminate (or continuous web 902) has a total thickness of about 0.08 mm (± 8 %). It is understood that the first and second layers 906, 908 can comprise any suitable materials and have any suitable thicknesses. For example, in one embodiment, one of the layers can be an oriented polypropylene (OPP) and the other layer can be a low-density polyethylene (LDPE).

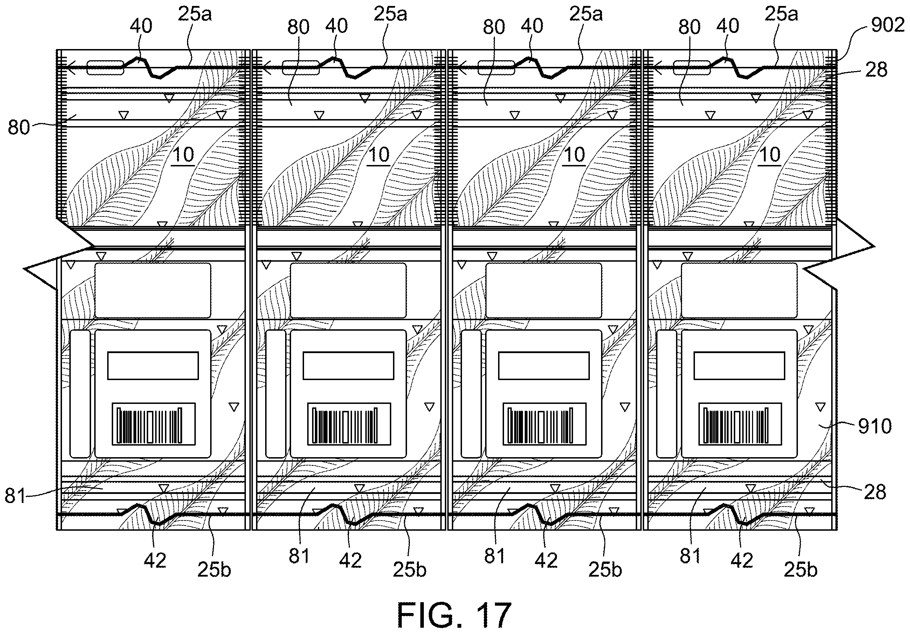

As seen in Figure 17, various printing elements 910 disposed on the continuous web 902 are readily visible when the first layer 906 is viewed by the consumer (or user). In the embodiment illustrated in Figure 17, the printing elements 910 include various patterns, graphics, and indicia (e.g., warnings, instructions of use, sku numbers and bars, brand advertisement). It is contemplated that the printing elements 910 can include any printing suitable for the bag 10 containing absorbent articles 60. In one suitable embodiment, the printing elements 910 are located on the inner surface (i.e., one of the bonded surfaces as seen in Figure 18) of at least one of the first and second layers 906, 908 and the first layer is generally transparent or sufficiently translucent such that the printing is readily visible by the consumer through the first layer. It is contemplated, however, that in other suitable embodiments the printing elements 910 can be located on the outer surface of the first layer 906. Additionally, indicia or guide words (not shown in Figure 17) can be disposed on the continuous web 902 adjacent resealable features 28 to assist the user in resealing the bag 10. In one suitable example, the resealable feature 28 can be made from LDPE. It is understood, however, that any suitable material that is compatible with the bag 10 material can be used to make the resealable feature 28. It is further understood that the resealable feature 28 can have any suitable configuration including, for example, multiple lanes, varying strengths, various flange heights, various weights, and various colors.

With reference again to Figure 16, the continuous web 902 is fed from the supply roll 904 to a laser station 912 for forming at least one of the individual separating seams 25a, 25b on the front and back walls 80, 81 of the bag 10. In the illustrated embodiment, the laser station 912 is adapted for forming both of the separating seams 25a, 25b. As mentioned above, the separating seams 25a, 25b can include perforations, weakened areas, and combinations of the two. The method depicted in Figures 16-18 illustrates forming weakened areas in the continuous web 902 in the form of a continuous line of weakness but it is understood that the method can be used to form perforations including perforated dashes, perforated lines and/or perforated dots or both perforations and weakened areas.

A pair of lasers 914 (one laser being seen in Figure 18) direct (or focus) a laser beam 917 at the first layer 906 of the continuous web 902 to melt the first layer and thereby form a "cut" through the first layer. In one suitable example, the first layer 906 has a melting point less than the melting point of the second layer 908. As a result, the lasers 914 can be selectively set to melt and thereby cut through the first layer 906 without melting/cutting the second layer 908. The lasers 914 (only one of the lasers being illustrated in Figure 18) are configured to cut entirely through the first layer 906. The second layer 908, in this example, is not cut or otherwise affected by the lasers 914. It is understood, however, that the second layer 908 could be cut by the lasers 914 and the first layer 906 could be unaffected by the lasers. It is further understood, that the lasers 914 can be selectively controlled to cut through any desired thickness of the continuous web 902. It is also understood that the laser can be selectively controlled to control the width of the cut. Thus, the cut can have any suitable width.

Referring again to Figure 17, which is a top plan view of the continuous web 902 exiting the laser station 912, the continuous web comprises a plurality of interconnected bags 10 being fed in the cross-machine direction. It is contemplated, however, that the interconnected bags 10 could be fed from the supply roll 904 in the machine direction. As illustrated in Figure 17, one of the pair of the lasers 914 forms the separating seams 25a and the associated tab 40 on the front wall 80, and the other laser forms the separating seam 25b and the associated tab 42 of the back wall 81. In the illustrated embodiment, both of the lasers of the pair of lasers 914 operate simultaneously and follow the same cutting path. That is, the lasers 914 follow identical paths such that the separating seams 25a and the associated tab 40 on the front wall 80 are the same as the separating seam 25b and the associated tab 42 of the back wall 81 as viewed in Figure 17. It is contemplated that a single laser can be used to form both of the separating seams 25a, 25b by passing the continuous web 902 by the laser twice. It is further contemplated that the separating seams 25a, 25b and/or tabs 40, 42 can have different configurations.

With reference again to Figure 16, the continuous web 902 moves from the laser station 912 to a cutting station 916 where the continuous web is cut in the cross machine direction to separate the web into discrete segments 918. The cutting station 916 can include any suitable cutting apparatus (e.g., a knife and anvil). It is contemplated that the cutting station 916 could be located before the laser station 912 such that the pair of lasers 914 act on the discrete segments 918. It is further contemplated that the discrete segments 918 can be directed to the laser station 912 from a suitable source thereby eliminating the continuous web 902 and the need for the cutting station 916.

In the illustrated embodiment, the discrete segments 918 are transferred from the cutting station 916 to a filling, folding, and sealing station 919 where the discrete segments 918 are folded into the desired bag shape, at least one of the articles 60 is placed in the bag 10 and the edges of the bags are sealed to enclose the article therein. As mentioned above with respect to the example of the bag of Figures 5 and 5A, eight digital tampons are enclosed within the bag 10. From the filling, folding, and sealing station 919, a plurality of the bags 10 containing at least one of the articles 60 can be boxed (or otherwise packaged) for shipping.

It is contemplated that the laser station 912 can be disposed at any suitable position during the process of manufacturing the bags 10 including after the cutting station such that the separating seams 25a, 25b are formed on the discrete segments 918, or after the filling, folding, and sealing station such that the separating seams 25a, 25b are formed on the sealed bags 10.

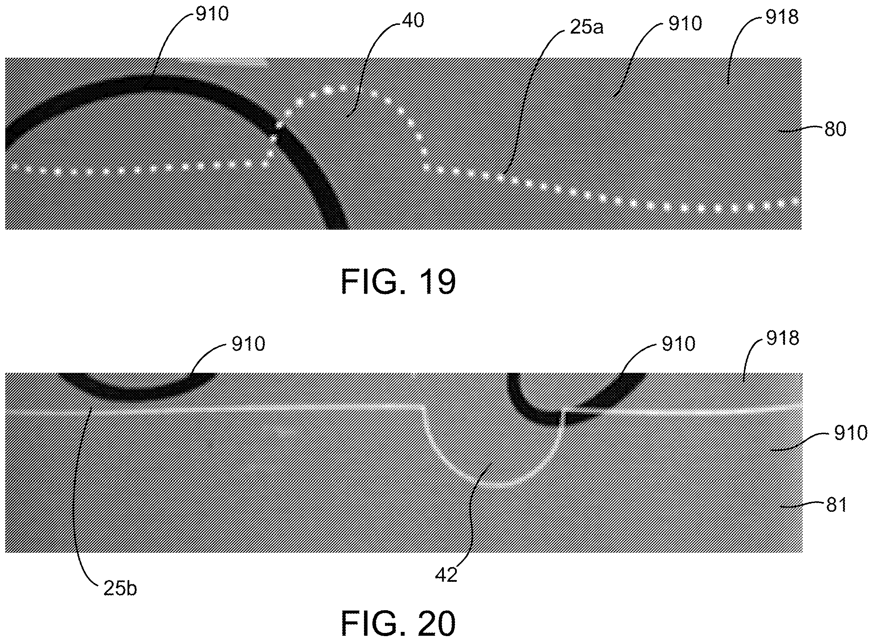

Figure 19 is a photograph of a portion of a discrete segment 918 having the separating seam 25a and the associated tab 40 formed thereon using a targeted laser scribing technique. In this embodiment, the separating seam 25a is defined by a plurality of generally circular perforations (i.e., perforation dots) that extend entirely through the thickness of the discrete segment 918. Figure 20 is a photograph of a portion of a discrete segment 918 having the separating seam 25b and the associated tab 42 formed thereon using a targeted laser scribing technique. In this embodiment, the separating seam 25b is defined by a continuous line of weakness that extends through a suitable portion of the thickness of the discrete segment 918. As readily seen in Figure 20, the printing elements 910, such as the printed background and black lines extending over the background, disposed on the segment 918 are at least partially removed (or otherwise altered) during the laser cutting process and thus acts as a visual cue to the consumer. As a result, the separating seam 25b is readily apparent to the consumer.

It is contemplated that the laser can be used to cut either the outside surface of the bag 10, as seen in Figures 16-20, or the inside surface of the bag. One potential advantage of cutting a line of weakness on the inside surface of the bag 10 to define the separating seams 25a, 25b and associated tabs 40, 42 is that the exterior of the bag remains unaltered and therefore appears to the consumer to be fully sealed.

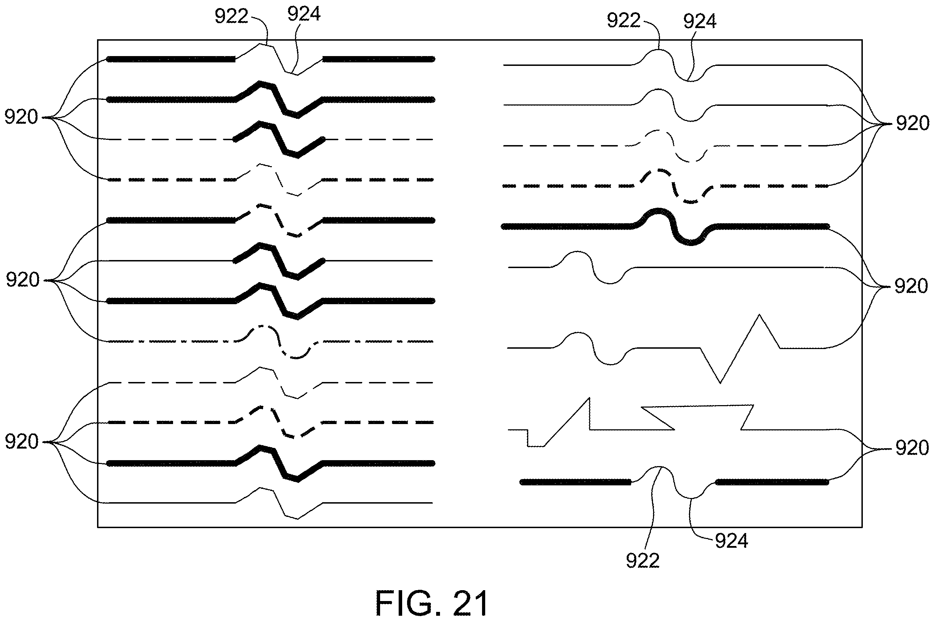

Figure 21 shows various suitable cutting patterns 920 for forming the separating seams 25a, 25b, and the associated tabs 40, 42, with a targeted laser scribing technique. Suitable targeted laser scribing techniques are disclosed in an article by William R. Dinauer et al. entitled CO2 Lasers for Flexible Food Packaging, www.laser-journal.de, pages 45 and 46, January 2008; and an article by David Hennig entitled Scoring Big with Lasers: Lasers and Packaging Technology which published January 2001 Issue of Flexible Packaging Magazine. The lasers 914 of laser station 912 can follow the various patterns 920 to form the separating seams 25a, 25b. Advantageously, the precision of the described laser scribing technique allows for use of an unlimited number of patterns to precisely shape the separating seams 25a, 25b and the associated tabs 40, 42; thus, the laser scribing technique is not limited to use of the patterns 920 shown in Figure 21. The patterns 920 provide for the lasers 914 to form separating seams 25a, 25b having continuous weakened lines, perforated dashes, extended perforated dashes, curved perforated dashes, perforated dots, other suitable weakened areas, and combinations of these. The separating seams 25a, 25b formed by following the patterns 920 can have combinations of straight portions, curved portions, and slanted portions and will define tabs 40, 42 having various geometric shapes, e.g., tabs with polygonal shapes, circular shapes, and triangular shapes. Additionally, some patterns 920 have varying widths along their length so the lasers 914 following these patterns 920 will form separating seams 25a, 25b having varying widths along their length. Some patterns 920 also enable the lasers 914 to form individual separating seams 25a, 25b that each define multiple tabs 40, 42. It is contemplated that the laser station 912 can include any suitable type and number of lasers 914 and associated equipment including, but not limited to, mirrors, pointers, and optics. For example, suitable lasers and laser stations are described in U.S. Patent Application Publication No. 2003/0047695 entitled "System and Method for Synchronizing a Laser Beam to a Moving Web" to Zik et al. and U.S. Patent No. 5,630,308 entitled "Laser Scoring of Packaging Substrates" to Guckenberger.

Some of the patterns 920 seen in Figure 21 have peaks 922 and valleys 924, which, beneficially, allow the formation of separating seams 25a, 25b defining valleys adjacent the tabs 40, 42. The adjacent valleys make the tabs 40, 42 more identifiable and allow the user to more easily grasp the tabs. However, in other suitable embodiments, the patterns may include only peaks for forming separating seams 25a, 25b that define tabs 40, 42 without associated valleys, or include peaks with multiple adjacent valleys for forming separating seams 25a, 25b that define tabs with multiple associated valleys, e.g., a tab with a valley on each side.

The various patterns 920 allow a manufacturer to select desired designs for forming the separating seams 25a, 25b and tabs 40, 42. For example, the manufacturer can select one of the patterns 920 that provides for forming a separating seam 25a, 25b with only straight and slanted portions to simplify the manufacturing process. Alternately, the manufacturer can select one of the patterns 920 that provides for forming a separating seam 25a, 25b with curved portions to produce tabs 40, 42 that are aesthetically pleasing and prevent tear off at corner stress points.

As various changes could be made in the above constructions without departing from the scope of the invention, it is intended that all matter contained in the above description and shown in the accompanying drawings shall be interpreted as illustrative and not in a limiting sense.

This written description uses examples to disclose the invention, including the best mode, and also to enable any person skilled in the art to practice the invention, including making and using any devices or systems and performing any incorporated methods. The patentable scope of the invention is defined by the claims.

When introducing elements of the present invention or the preferred embodiment(s) thereof, the articles "a", "an", "the" and "said" are intended to mean that there are one or more of the elements. The terms "comprising", "including" and "having" are intended to be inclusive and mean that there may be additional elements other than the listed elements.

## Claims

1. A method for forming a resealable and tamper resistant bag (10) for enclosing at least one absorbent article (60) comprising:
moving a planar substrate in a machine direction, the planar substrate having two opposite edges;
attaching a first resealable feature to the substrate generally adjacent one of the edges;
attaching a second resealable feature to the substrate in spaced relationship to the first resealable feature and generally adjacent the other edge, the first and second resealable features (28) being configured to sealingly engage;
forming a first separating seam (25a) on the substrate between the first resealable feature and the respective edge of the substrate, the first separating seam (25a) defining a first tab (40);
forming a second separating seam (25b) on the substrate between the second resealable feature and the respective edge such that the first and second separating seams (25a, 25b) are generally the same distance from the respective first and second resealable features (28), the second separating seam (25b) defining a second tab (42), the second tab (42) being offset from the first tab (40);
placing at least one absorbent article (60) in contact with the substrate; and
folding and sealing the substrate to define the resealable and tamper resistant bag (10) having the at least one absorbent article (60) enclosed therein.

2. The method of claim 1 wherein forming at least one and preferably both of the first and second separating seams (25a, 25b) comprises forming the separating seam (25) using a targeted laser scribing technique wherein at least one laser is used to cut the substrate.

3. The method of claim 2 wherein the targeted laser scribing technique comprises a pair of lasers (914) and both the first (25a) and second (25b) separating seams are formed simultaneously by the lasers (914).

4. A method for forming a plurality of resealable and tamper resistant bags (10) for enclosing absorbent articles (60) therein, the method comprising:
directing, in a machine direction, a continuous web (902) comprising a plurality of interconnected segments suitable for the construction of a plurality of the bags (10), the plurality of segments being interconnected in the cross-machine direction;
attaching a first resealable feature to the continuous web (902);
attaching a second resealable feature to the web (902) in spaced relationship to the first resealable feature;
forming at least one continuous separating seam on the web using a laser, the at least one separating seam extending across the plurality of interconnected segments and defining at least one tab (40) on each of the segments;
cutting the continuous web (902) in the cross-machine direction to separate the plurality of interconnected segments from the web to form a plurality of discrete segments (918);
associating at least one absorbent article (60) with each discrete segment (918); and folding and sealing each of the discrete segments (918) to enclose the at least one absorbent article (60).

5. The method of claim 4 wherein forming the at least one continuous separating seam on the web (902) comprises forming two, spaced-apart separating seams (25a, 25b) using a pair of lasers (914), each laser forming a different seam, wherein each of the lasers defines a cutting path, the cutting path of both lasers (914) being preferably the same.

6. The method of claim 4 wherein the continuous web (902) comprises a laminate having first (906) and second layers (908), and wherein forming the at least one continuous separating seam comprises cutting the first layer (906) of the web (902) using the laser by melting the first layer (906).

7. The method of claim 6 wherein the at least one tab on each of the segments comprises a folded tab (504) structure.

8. The method of claim 7 wherein folding and sealing each of the discrete segments (918) comprises engaging the first and second resealable features (28).

9. The method of claim 7 wherein folding and sealing each of the discrete segments (918) comprising folding each of the discrete segments (918) generally in half to form a front wall (80) and a back wall (81) of the bag (10), wherein folding and sealing each of the discrete segments (918) comprising bonding at least a portion of the front wall (80) to at least a portion of the back wall (81) to form a holding portion (20) of the bag (10).

10. The method of any preceding claim wherein the pair of resealable features (28) are selected from a group consisting of interlocking groove and rib structures, adhesive strips, hook and loop structures, materials having varying coefficients of friction, and zipper-like features.

11. The method of any preceding claim wherein the separating seams are selected from a group consisting of perforations, weakened areas, and combinations of the two.

12. The method of any preceding claim, further comprising supporting the tabs (40; 42) with a strengthening agent selected from a group consisting of a strengthening coating, additional basis weight of tab forming material, and adhesive patches of strengthening material.

13. The method of claim 11, wherein the separating seams are provided by perforations and the tabs (40; 42) are defined by one or more extended perforations which traverse at least a portion of the periphery of the tabs (40; 42).

## Patentansprüche

1. Verfahren für das Ausbilden eines wiederverschließbaren und manipulationssicheren Beutels (10) für das Einschließen mindestens eines absorbierenden Artikels (60), umfassend:
Bewegen eines planaren Substrats in eine Maschinenrichtung, wobei das planare Substrat zwei gegenüberliegende Kanten hat;
Befestigen eines ersten wiederverschließbaren Merkmals am Substrat im Wesentlichen neben einer der Kanten;
Befestigen eines zweiten wiederverschließbaren Merkmals am Substrat in beabstandeter Beziehung zum ersten wiederverschließbaren Merkmal und im Allgemeinen neben der anderen Kante, wobei die ersten und zweiten wiederverschließbaren Merkmale (28) so ausgestaltet sind, dass sie im versiegelnden Eingriff sind;
Ausbilden einer ersten Trennnaht (25a) am Substrat zwischen dem ersten wiederverschließbaren Merkmal und der jeweiligen Kante des Substrats, wobei die erste Trennnaht (25a) eine erste Lasche (40) definiert;
Ausbilden einer zweiten Trennnaht (25b) am Substrat zwischen dem zweiten wiederverschließbaren Merkmal und der jeweiligen Kante, sodass die ersten und zweiten Trennnähte (25a, 25b) im Allgemeinen denselben Abstand von den jeweiligen ersten und zweiten wiederverschließbaren Merkmalen (28) haben, wobei die zweite Trennnaht (25b) eine zweite Lasche (42) definiert, wobei die zweite Lasche (42) von der ersten Lasche (40) versetzt ist;
Platzieren mindestens eines absorbierenden Artikels (60) in Kontakt mit dem Substrat; und
Falten und Versiegeln des Substrats, um den wiederverschließbaren und manipulationssicheren Beutel (10) mit mindestens einem absorbierenden Artikel (60) darin eingeschlossen auszubilden.

2. Verfahren nach Anspruch 1, worin das Ausbilden mindestens einer und bevorzugt sowohl der ersten als auch der zweiten Trennnähte (25a, 25b) das Ausbilden der Trennnaht (25) mithilfe einer zielgerichteten Laserritztechnik umfasst, wobei mindestens ein Laser zum Schneiden des Substrats verwendet wird.

3. Verfahren nach Anspruch 2, worin die zielgerichtete Laserritztechnik ein Paar Laser (914) umfasst und sowohl die ersten (25a) als auch die zweiten (25b) Trennnähte gleichzeitig durch die Laser (914) ausgebildet werden.

4. Verfahren für das Ausbilden einer Vielzahl von wiederverschließbaren und manipulationssicheren Beuteln (10) für das Einschließen absorbierender Artikel (60) darin, das Verfahren umfassend:
Leiten, in eine Maschinenrichtung, einer kontinuierlichen Bahn (902), die eine Vielzahl von miteinander verbundenen Segmenten umfasst, die für die Konstruktion einer Vielzahl von Beuteln (10) geeignet sind, wobei die Vielzahl von Segmenten in Maschinenquerrichtung miteinander verbunden sind;
Befestigen eines ersten wiederverschließbaren Merkmals an der kontinuierlichen Bahn (902);
Befestigen eines zweiten wiederverschließbaren Merkmals an der Bahn (902) in beabstandeter Beziehung zum ersten wiederverschließbaren Merkmal;
Ausbilden mindestens einer kontinuierlichen Trennnaht an der Bahn mithilfe eines Lasers, wobei die mindestens eine Trennnaht sich über die Vielzahl von miteinander verbundenen Segmenten erstreckt und mindestens eine Lasche (40) an jedem der Segmente definiert;
Schneiden der kontinuierlichen Bahn (902) in der Maschinenquerrichtung, um die Vielzahl von miteinander verbundenen Segmenten von der Bahn zu trennen, um eine Vielzahl von einzelnen Segmenten (918) auszubilden;
Zuordnen mindestens eines absorbierenden Artikels (60) zu jedem einzelnen Segment (918); und Falten und Versiegeln jedes der einzelnen Segmente (918), um den mindestens einen absorbierenden Artikel (60) einzuschließen.

5. Verfahren nach Anspruch 4, worin das Ausbilden der mindestens einen kontinuierlichen Trennnaht an der Bahn (902) das Ausbilden von zwei, beabstandeten Trennnähten (25a, 25b) mithilfe eines Laserpaars (914) umfasst, wobei jeder Laser eine andere Naht ausbildet, worin jeder der Laser einen Schneidpfad definiert, wobei der Schneidpfad beider Laser (914) bevorzugt derselbe ist.

6. Verfahren nach Anspruch 4, worin die kontinuierliche Bahn (902) ein Laminat umfasst, das erste (906) und zweite Schichten (908) aufweist, und worin das Ausbilden der mindestens einen kontinuierlichen Trennnaht das Schneiden der ersten Schicht (906) der Bahn (902) mithilfe des Lasers durch Schmelzen der ersten Schicht (906) umfasst.

7. Verfahren nach Anspruch 6, worin die mindestens eine Lasche an jedem der Segmente eine gefaltete Laschen(504)-Struktur umfasst.

8. Verfahren nach Anspruch 7, worin das Falten und Versiegeln jedes der einzelnen Segmente (918) das in Eingriff bringen der ersten und zweiten wiederverschließbaren Merkmale (28) umfasst.

9. Verfahren nach Anspruch 7, worin das Falten und Versiegeln jedes der einzelnen Segmente (918) das Falten jedes der einzelnen Segmente (918) im Allgemeinen in der Mitte umfasst, um eine vordere Wand (80) und eine hintere Wand (81) des Beutels (10) auszubilden, worin das Falten und Versiegeln jedes der einzelnen Segmente (918) das Bonden mindestens eines Abschnitts der vorderen Wand (80) mit mindestens einem Abschnitt der hinteren Wand (81) umfasst, um einen Halteabschnitt (20) des Beutels (10) auszubilden.

10. Verfahren nach einem der vorstehenden Ansprüche, worin das Paar wiederverschließbare Merkmale (28) aus einer Gruppe ausgewählt wird, die aus ineinandergreifenden Rillen- und Rippenstrukturen, Klebstreifen, Klettbandstrukturen, Materialien mit unterschiedlichen Reibungskoeffizienten und reißverschlussähnlichen Merkmalen besteht.

11. Verfahren nach einem der vorstehenden Ansprüche, worin die Trennnähte aus einer Gruppe ausgewählt werden, die aus Perforationen, geschwächten Bereichen und Kombinationen von beiden besteht.

12. Verfahren nach einem der vorstehenden Ansprüche, ferner umfassend das Tragen der Laschen (40; 42) mit einem Verstärkungsmittel, das aus einer Gruppe ausgewählt wird, die aus einer Verstärkungsbeschichtung, zusätzlichem Flächengewicht des laschenausbildenden Materials und Klebeflecken aus Verstärkungsmaterial besteht.

13. Verfahren nach Anspruch 11, worin die Trennnähte durch Perforationen bereitgestellt werden und die Laschen (40; 42) durch eine oder mehrere erweiterte Perforationen definiert werden, die mindestens einen Abschnitt der Peripherie der Laschen (40; 42) queren.

## Revendications

1. Procédé de formation d'un sac refermable et inviolable (10) destiné à renfermer au moins un article absorbant (60), comprenant :
le déplacement d'un substrat plan dans un sens machine, le substrat plan ayant deux bords opposés ;
la fixation d'un premier élément refermable au substrat généralement à côté de l'un des bords ;
la fixation d'un deuxième élément refermable au substrat en relation espacée avec le premier élément refermable et généralement à côté de l'autre bord, les premier et deuxième éléments refermables (28) étant conçus pour venir en prise de manière étanche ;
la formation d'une première couture de séparation (25a) sur le substrat entre le premier élément refermable et le bord respectif du substrat, la première couture de séparation (25a) définissant une première languette (40) ;
la formation d'une deuxième couture de séparation (25b) sur le substrat entre le deuxième élément refermable et le bord respectif de sorte que les première et deuxième coutures de séparation (25a, 25b) se trouvent généralement à la même distance des premier et deuxième éléments refermables (28) respectifs, la deuxième couture de séparation (25b) définissant une deuxième languette (42), la deuxième languette (42) étant décalée de la première languette (40) ;
le placement d'au moins un article absorbant (60) en contact avec le substrat, et
le pliage et le scellement du substrat pour définir le sac refermable et inviolable (10) renfermant l'au moins un article absorbant (60).

2. Procédé de la revendication 1, dans lequel la formation d'au moins une et de préférence des deux des première et deuxième coutures de séparation (25a, 25b) comprend la formation de la couture de séparation (25) à l'aide d'une technique de découpe laser ciblée dans laquelle au moins un laser est utilisé pour découper le substrat.

3. Procédé de la revendication 2, dans lequel la technique de découpe laser ciblée comprend une paire de lasers (914) et les deux des première (25a) et deuxième (25b) coutures de séparation sont formées simultanément par les lasers (914).

4. Procédé de formation d'une pluralité de sacs refermables et inviolables (10) destinés à renfermer des articles absorbants (60), le procédé comprenant :
la direction, dans un sens machine, d'une bande continue (902) comprenant une pluralité de segments raccordés entre eux appropriés pour la construction d'une pluralité de sacs (10), la pluralité de segment étant raccordés entre eux dans le sens travers ;
la fixation d'un premier élément refermable à la bande continue (902) ;
la fixation d'un deuxième élément refermable à la bande (902) en relation espacée avec le premier élément refermable ;
la formation d'au moins une couture de séparation continue sur la bande à l'aide d'un laser, l'au moins une couture de séparation s'étendant sur la pluralité de segments raccordés entre eux et définissant au moins une languette (40) sur chacun des segments ;
la découpe de la bande continue (902) dans le sens travers pour séparer la pluralité de segments raccordés entre eux de la bande pour former une pluralité de segments discrets (918) ;
l'association d'au moins un article absorbant (60) à chaque segment discret (918) ; et le pliage et le scellement de chacun des segments discret (918) pour renfermer l'au moins un article absorbant (60).

5. Procédé de la revendication 4, dans lequel la formation de l'au moins une couture de séparation continue sur la bande (902) comprend la formation de deux coutures de séparation espacées (25a, 25b) à l'aide d'une paire de lasers (914), chaque laser formant une couture différente, chacun des lasers définissant un trajet de coupe, le trajet de coupe des deux lasers (914) étant de préférence le même.

6. Procédé de la revendication 4, dans lequel la bande continue (902) comprend un stratifié ayant des première (906) et deuxième (908) couches, et dans lequel la formation de l'au moins une couture de séparation continue comprend la découpe de la première couche (906) de la bande (902) à l'aide du laser par fusion de la première couche (906).

7. Procédé de la revendication 6, dans lequel l'au moins une languette sur chacun des segments comprend une structure de languette pliée (504).

8. Procédé de la revendication 7, dans lequel le pliage et le scellement des segments discrets (918) comprend la mise en prise des premier et deuxième éléments refermables (28).

9. Procédé de la revendication 7, dans lequel le pliage et le scellement de chacun des segments discrets (918) comprend le pliage de chacun des segments discrets (918) généralement en deux pour former une paroi avant (80) et une paroi arrière (81) du sac (10), le pliage et le scellement de chacun des segments discrets (918) comprenant la liaison d'au moins une partie de la paroi avant (80) à au moins une partie de la paroi arrière (81) pour former une partie de contenance (20) du sac (10).

10. Procédé de l'une quelconque des revendications précédentes, dans lequel la paire d'éléments refermables (28) est choisi dans un groupe constitué par des structures de rainures et nervures, des bandes adhésives, des structures de crochets et boucles, de matériaux ayant des coefficients de frottement variables, et des éléments de type fermeture à glissière.

11. Procédé de l'une quelconque des revendications précédentes, dans lequel les coutures de séparation sont choisies dans un groupe constitué par des perforations, des zones de faiblesse et des combinaisons de ces deux éléments.

12. Procédé de l'une quelconque des revendications précédentes, comprenant en outre le support des languettes (40 ; 42) avec un agent de renfort choisi dans un groupe constitué par un revêtement de renfort, un poids de base supplémentaire de matériau de formation de languette et des pièces adhésives de matériau de renfort.

13. Procédé de la revendication 11, dans lequel les coutures de séparation sont formées par des perforations et les languettes (40 ; 42) sont définies par au moins une perforation étendue qui traverse au moins une partie de la périphérie des languettes (40 ; 42).
